# EUROPEAN PATENT APPLICATION

(11) **EP 3 581 586 A1**
(43) Date of publication of application: **18.12.2019**
(21) Application number: 19161510.3
(22) Date of filing: 23.04.2015
(51) Int. Cl.: C07K 16/32, A61K 47/68, A61K 31/513, A61K 31/5365, A61K 31/675, A61K 31/704, A61K 39/395, A61K 39/00

(54) **METHODS OF TREATING EARLY BREAST CANCER WITH TRASTUZUMAB-MCC-DM1 AND PERTUZUMAB**

(30) Priority: 25.04.2014 US 201461984132 P
(62) Divisional of application: 15720875.2
(71) Applicant: F. Hoffmann-La Roche AG, 4070 Basel (CH)
(72) Inventor: Green, Marjorie C., South San Francisco, California 94080 (US); Guardino, Alice Elizabeth, South San Francisco, California 94080 (US)
(74) Representative: Klostermeyer-Rauber, Dörte

(57) **Abstract**

Methods of treating patients having HER2-positive, operable, locally advanced or inflammatory breast cancer with the antibody-drug conjugate Trastuzumab-MCC-DM1 and Pertuzumab are provided.

## Description

### RELATED APPLICATIONS

The present application claims benefit under 35 U.S.C. § 119 of U.S. Provisional Patent Application No. 61/984,132, filed on April 25, 2014, the disclosure of which is hereby incorporated by reference in its entirety.

### SEQUENCE LISTING

The instant application contains a Sequence Listing in ASCII format and is hereby incorporated by reference in its entirety. The ASCII text file was created on April xx, 2015, is named GNE-0412WO_SL.txt and is xxxxx bytes in size.

### FIELD OF THE INVENTION

The invention relates to methods of using Trastuzumab-MCC-DMl and Pertuzumab for the treatment of early breast cancer (EBC).

**BACKGROUND OF THE INVENTION**

### Breast Cancer and HER2 Targeted Treatments

Breast cancer is a highly significant cause of morbidity and mortality worldwide. There are over 1.3 million cases of breast cancer diagnosed globally each year with more than 450,000 deaths related to the disease (Jemal A, Bray F, Center M, et al. Global cancer statistics. CA Cancer J Clin, 2011; 61(2):69-90).

The HER2 (ErbB2) receptor tyrosine kinase is a member of the epidermal growth factor receptor (EGFR) family of transmembrane receptors. Overexpression of HER2 is observed in approximately 20% of human breast cancers (hereinafter referred to as HER2-positive breast cancer) and is implicated in the aggressive growth and poor clinical outcomes associated with these tumors (Slamon et al (1987) Science 235:177-182). HER2 protein overexpression can be determined using an immunohistochemistry based assessment of fixed tumor blocks (Press MF, et al (1993) Cancer Res 53:4960-70).

Trastuzumab (CAS 180288-69-1, HERCEPTIN®, huMAb4D5-8, rhuMAb HER2, Genentech) is a recombinant DNA-derived, IgG1 kappa, monoclonal antibody that is a humanized version of a murine anti-HER2 antibody (4D5) that selectively binds with high affinity in a cell-based assay (Kd = 5 nM) to the extracellular domain of HER2 (US 5677171; US 5821337; US 6054297; US 6165464; US 6339142; US 6407213; US 6639055; US 6719971; US 6800738; US 7074404; Coussens et al (1985) Science 230:1132-9; Slamon et al (1989) Science 244:707-12; Slamon et al (2001) New Engl. J. Med. 344:783-792). Trastuzumab has been shown, in both *in vitro* assays and in animals, to inhibit the proliferation of human tumor cells that overexpress HER2 (Hudziak et al (1989) Mol Cell Biol 9:1165-72; Lewis et al (1993) Cancer Immunol Immunother; 37:255-63; Baselga et al (1998) Cancer Res. 58:2825-2831). Trastuzumab is a mediator of antibody-dependent cellular cytotoxicity, ADCC (Lewis et al (1993) Cancer Immunol Immunother 37(4):255-263; Hotaling et al (1996) [abstract]. Proc. Annual Meeting Am Assoc Cancer Res; 37:471; Pegram MD, et al (1997) [abstract]. Proc Am Assoc Cancer Res; 38:602; Sliwkowski et al (1999) Seminars in Oncology 26(4), Suppl 12:60-70; Yarden Y. and Sliwkowski, M. (2001) Nature Reviews: Molecular Cell Biology, Macmillan Magazines, Ltd., Vol. 2:127-137).

HERCEPTIN® was approved in 1998 for the treatment of patients with HER2-overexpressing metastatic breast cancers (Baselga et al, (1996) J. Clin. Oncol. 14:737-744) that have received extensive prior anti-cancer therapy, and has since been used in over 300,000 patients (Slamon DJ, et al. N Engl J Med 2001;344:783-92; Vogel CL, et al. J Clin Oncol 2002;20:719-26; Marty M, et al. J Clin Oncol 2005;23:4265-74; Romond EH, et al. T N Engl J Med 2005;353:1673-84; Piccart-Gebhart MJ, et al. N Engl J Med 2005;353:1659-72; Slamon D, et al. [abstract]. Breast Cancer Res Treat 2006, 100 (Suppl 1): 52). In 2006, the FDA approved HERCEPTIN® (Trastuzumab, Genentech Inc.) as part of a treatment regimen containing doxorubicin, cyclophosphamide and paclitaxel for the adjuvant treatment of patients with HER2-positive, node-positive breast cancer.

An alternative approach to antibody-targeted therapy is to utilize antibodies for delivery of cytotoxic drugs specifically to antigen-expressing cancer cells. Antibody-drug conjugates, or ADCs, are monoclonal antibodies to which highly potent cytotoxic agents have been conjugated. ADCs represent a novel approach to conferring tumor selectivity on systemically administered anti-tumor therapeutics. Utilizing surface antigens that are tumor-specific and/or overexpressed, ADCs are designed to focus the delivery of highly potent cytotoxic agents to tumor cells. The potential of this approach is to create a more favorable therapeutic window for such agents than could be achieved by their administration as free drugs.

Maytansinoids, derivatives of the anti-mitotic drug maytansine, bind to microtubules in a manner similar to vinca alkaloid drugs (Issell BF et al (1978) Cancer Treat. Rev. 5:199-207; Cabanillas F et al. (1979) Cancer Treat Rep, 63:507-9. DM1 is a thiol-containing maytansinoid derived from the naturally occurring ester ansamitocin P3 (Remillard S, Rebhun LI, Howie GA, et al. (1975) Science 189(4207):1002-1005.3; Cassady JM, Chan KK, Floss HG. (2004) Chem Pharm Bull 52(1):1-26.4). The related plant ester, maytansine, has been studied as a chemotherapeutic agent in approximately 800 patients, administered at a dose of 2.0 mg/m2 every 3 weeks either as a single dose or for 3 consecutive days (Issell BF, Crooke ST. (1978) Maytansine. Cancer Treat Rev 5:199-207). Despite preclinical activity, the activity of maytansine in the clinic was modest at doses that could be safely delivered. The dose-limiting toxicity (DLT) was gastrointestinal, consisting of nausea, vomiting, and diarrhea (often followed by constipation). These toxicities were dose dependent but not schedule dependent. Peripheral neuropathy (predominantly sensory) was reported and was most apparent in patients with preexisting neuropathy. Subclinical transient elevations of hepatic transaminase, alkaline phosphatase, and total bilirubin were reported. Constitutional toxicities, including weakness, lethargy, dysphoria, and insomnia, were common. Less common toxicities included infusion-site phlebitis and mild myelosuppression. Further development of the drug was abandoned in the 1980s because of the narrow therapeutic window.

Trastuzumab-MCC-DMl (T-DM1, Trastuzumab emtansine, ado-Trastuzumab emtansine, KADCYLA®), a novel antibody-drug conjugate (ADC) for the treatment of HER2-positive breast cancer, is composed of the cytotoxic agent DM1 (a thiol-containing maytansinoid anti-microtubule agent) conjugated to Trastuzumab at lysine side chains via an MCC linker, with an average drug load (drug to antibody ratio) of about 3.5. After binding to HER2 expressed on tumor cells, T-DM1 undergoes receptor-mediated internalization, resulting in intracellular release of cytotoxic catabolites containing DM1 and subsequent cell death.

In a Phase I study of T-DM1 (TDM3569g), the maximum tolerated dose (MTD) of T-DM1 administered by IV infusion every 3 weeks (q3w) was 3.6 mg/kg. A DLT (Dose-Limiting Toxicity) consisted of transient thrombocytopenia in patients treated at 4.8 mg/kg. Treatment with 3.6 mg/kg q3w was well tolerated and associated with significant clinical activity. (Krop (2010) J. Clin. Oncol. 28(16):2698-2704). That same study also showed that weekly dosing with 2.4 mg/kg was also well tolerated and had anti-tumor activity. (Beeram (2012) Cancer 118(23):5733-5740.)

A Phase II study (TDM4374g) demonstrated that T-DM1, administered at 3.6 mg/kg q3w, had single-agent anti-tumor activity in a heavily pre-treated patient population having HER2-positive metastatic breast cancer. (Krop (2012) 30(26):3234-3241.) A Phase III study (TDM4370g) demonstrated that T-DM1, administered at 3.6 mg/kg q3w, significantly prolonged progression-free survival and overall survival with less toxicity compared to treatment with lapatinib plus capecitabine in patients with HER2-positive advanced breast cancer previously treated with Trastuzumab and a taxane. (Verma (2012) New England Journal of Medicine 367:1783-1791.)

The U.S. Food and Drug Administration approved ado-Trastuzumab emtansine, marketed under the tradename KADCYLA®, on February 22, 2013 for the treatment of patients with HER2-positive, metastatic breast cancer who previously received treatment with Trastuzumab and a taxane.

Pertuzumab (also known as recombinant humanized monoclonal antibody 2C4, rhuMAb 2C4, PERJETA®, Genentech, Inc, South San Francisco) represents the first in a new class of agents known as HER dimerization inhibitors (HDI) and functions to inhibit the ability of HER2 to form active heterodimers or homodimers with other HER receptors (such as EGFR/HER1, HER2, HER3 and HER4). See, for example, Harari and Yarden Oncogene 19:6102-14 (2000); Yarden and Sliwkowski. Nat Rev Mol Cell Biol 2:127-37 (2001); Sliwkowski Nat Struct Biol 10:158-9 (2003); Cho et al. Nature 421:756-60 (2003); and Malik et al. Pro Am Soc Cancer Res 44:176-7 (2003)

Pertuzumab blockade of the formation of HER2-HER 3 heterodimers in tumor cells has been demonstrated to inhibit critical cell signaling, which results in reduced tumor proliferation and survival (Agus et al. Cancer Cell 2:127-37 (2002)).

Pertuzumab has undergone testing as a single agent in the clinic with a phase Ia trial in patients with advanced cancers and phase II trials in patients with ovarian cancer and breast cancer as well as lung and prostate cancer. In a Phase I study, patients with incurable, locally advanced, recurrent or metastatic solid tumors that had progressed during or after standard therapy were treated with Pertuzumab given intravenously every 3 weeks. Pertuzumab was generally well tolerated. Tumor regression was achieved in 3 of 20 patients evaluable for response. Two patients had confirmed partial responses. Stable disease lasting for more than 2.5 months was observed in 6 of 21 patients (Agus et al. Pro Am Soc Clin Oncol 22:192 (2003)). At doses of 2.0-15 mg/kg, the pharmacokinetics of Pertuzumab was linear, and mean clearance ranged from 2.69 to 3.74 mL/day/kg and the mean terminal elimination half-life ranged from 15.3 to 27.6 days. Antibodies to Pertuzumab were not detected (Allison et al. Pro Am Soc Clin Oncol 22:197 (2003)).

US 2006/0034842 describes methods for treating ErbB-expressing cancer with anti-ErbB2 antibody combinations. US 2008/0102069 describes the use of Trastuzumab and Pertuzumab in the treatment of HER2-positive metastatic cancer, such as breast cancer. Baselga et al., J Clin Oncol, 2007 ASCO Annual Meeting Proceedings Part I, Col. 25, No. 18S (June 20 Supplement), 2007:1004 report the treatment of patients with pre-treated HER2-positive breast cancer, which has progressed during treatment with Trastuzumab, with a combination of Trastuzumab and Pertuzumab. Portera et al., J Clin Oncol, 2007 ASCO Annual Meeting Proceedings Part I. Vol. 25, No. 18S (June 20 Supplement), 2007:1028 evaluated the efficacy and safety of Trastuzumab+Pertuzumab combination therapy in HER2-positive breast cancer patients, who had progressive disease on Trastuzumab-based therapy. The authors concluded that further evaluation of the efficacy of combination treatment was required to define the overall risk and benefit of this treatment regimen.

Pertuzumab has been evaluated in Phase II studies in combination with Trastuzumab in patients with HER2-positive metastatic breast cancer who have previously received Trastuzumab for metastatic disease. One study, conducted by the National cancer Institute (NCl), enrolled 11 patients with previously treated HER2-positive metastatic breast cancer. Two out of the 11 patients exhibited a partial response (PR) (Baselga et al., J Clin Oncol 2007 ASCO Annual Meeting Proceedings; 25:18 S (June 20 Supplement): 1004. The results of a Phase II neoadjuvant study evaluating the effect of a novel combination regimen of Pertuzumab and Trastuzumab plus chemotherapy (Docetaxel) in women with early-stage HER2-positive breast cancer, presented at the CTRC-AACR San Antonio Breast Cancer Symposium (SABCS), Dec. 8-12, 2010, showed that the two HER2 antibodies plus Docetaxel given in the neoadjuvant setting prior to surgery significantly improved the rate of complete tumor disappearance (pathological complete response rate, pCR, of 45.8 percent) in the breast by more than half compared to Trastuzumab plus Docetaxel (pCR of 29.0 percent), p=0.014.

Pertuzumab, marketed under the tradename PERJETA®, was approved in 2012 for the treatment of patients with advanced or late-stage (metastatic) HER2-positive breast cancer. HER2-positive breast cancers have increased amounts of the HER2 protein that contributes to cancer cell growth and survival.

On September 30, 2013, the U.S. Food and Drug Administration granted accelerated approval to PERJETA® (Pertuzumab) as part of a complete treatment regimen for patients with early stage breast cancer (EBC) before surgery (neoadjuvant setting). PERJETA® is the first FDA-approved drug for the neoadjuvant treatment of breast cancer.

Patent Publications related to HER2 antibodies include: U.S. Pat. Nos. 5,677,171; 5,720,937; 5,720,954; 5,725,856; 5,770,195; 5,772,997; 6,165,464; 6,387,371; 6,399,063; 6,015,567; 6,333,169; 4,968,603; 5,821,337; 6,054,297; 6,407,213; 6,639,055;6,719,971; 6,800,738; 8,075,890; 5,648,237; 7,018,809; 6,267,958; 6,685,940; 6,821,515; 7,060,268; 7,682,609; 7,371,376; 6,127,526; 6,333,398; 6,797,814; 6,339,142; 6,417,335; 6,489,447; 7,074,404; 7,531,645; 7,846,441; 7,892,549; 8,075,892; 6,573,043; 6,905,830; 7,129,051; 7,344,840; 7,468,252; 7,674,589; 7,919,254; 6,949,245; 7,485,302; 7,498,030; 7,501,122; 7,537,931; 7,618,631; 7,862,817; 7,041,292; 6,627,196; 7,371,379; 6,632,979; 7,097,840; 7,575,748; 6,984,494; 7,279,287; 7,811,773; 7,993,834; 8,076,066; 8,044,017; 7,435,797; 7,850,966; 7,485,704; 7,807,799; 8,142,784; 7,560,111; 7,879,325; 8,241,630; 7,449,184; 8,163,287; 7,700,299; 7,981,418; 8,247,397; and US 2010/0016556; US 2005/0244929; US 2001/0014326; US 2003/0202972; US 2006/0099201; US 2010/0158899; US 2011/0236383; US 2011/0033460; US 2008/0286280; US 2005/0063972; US 2006/0182739; US 2009/0220492; US 2003/0147884; US 2004/0037823; US 2005/0002928; US 2007/0292419; US 2008/0187533; US 2011/0250194; US 2012/0034213; US 2003/0152987; US 2005/0100944; US 2006/0183150; US 2008/0050748; US 2009/0155803; US 2010/0120053; US 2005/0244417; US 2007/0026001; US 2008/0160026; US 2008/0241146; US 2005/0208043; US 2005/0238640; US 2006/0034842; US 2006/0073143; US 2006/0193854; US 2006/0198843; US 2011/0129464; US 2007/0184055; US 2007/0269429; US 2008/0050373; US 2006/0083739; US 2009/0087432; US 2006/0210561; US 2002/0035736; US 2002/0001587; US 2008/0226659; US 2002/0090662; US 2006/0046270; US 2008/0108096; US 2007/0166753; US 2008/0112958; US 2009/0239236; US 2012/0034609; US 2012/0093838; US 2004/0082047; US 2012/0065381; US 2009/0187007; US 2011/0159014; US 2004/0106161; US 2011/0117096; US 2004/0258685; US 2009/0148402; US 2009/0099344; US 2006/0034840; US 2011/0064737; US 2005/0276812; US 2008/0171040; US 2009/0202536; US 2006/0013819; US 2012/0107391; US 2006/0018899; US 2009/0285837; US 2011/0117097; US 2006/0088523; US 2010/0015157; US 2006/0121044; US 2008/0317753; US 2006/0165702; US 2009/0081223; US 2006/0188509; US 2009/0155259; US 2011/0165157; US 2006/0204505; US 2006/0212956; US 2006/0275305; US 2012/0003217; US 2007/0009976; US 2007/0020261; US 2007/0037228; US 2010/0112603; US 2006/0067930; US 2007/0224203; US 2011/0064736; US 2008/0038271; US 2008/0050385; US 2010/0285010; US 2011/0223159; US 2008/0102069; US 2010/0008975; US 2011/0245103; US 2011/0246399; US 2011/0027190; US 2010/0298156; US 2011/0151454; US 2011/0223619; US 2012/0107302; US 2009/0098135; US 2009/0148435; US 2009/0202546; US 2009/0226455; US 2009/0317387; US 2011/0044977; US 2012/0121586.

### HER2 Positive Early Stage Breast Cancer (EBC)

For early stage breast cancer (EBC), prognostic factors for relapse include: stage of disease including evidence of ability to spread, (i.e., lymphovascular invasion or lymphatic involvement) and molecular subtype. Tumors with more aggressive biology have increased risk of relapse, such as tumors that have any or all of the following: evidence of increased proliferative activity, higher nuclear grade, lower levels of hormone receptor expression and overexpression of HER2 (Ross J, Slodkowska E, Symmans W, et al.The HER-2 receptor and breast cancer: ten years of targeted anti-HER-2 therapy and personalized medicine. The Oncologist, 2009; 14(4):320-68; Mazouni C, Peintinger F, Wan-Kau S, et al. Residual ductal carcinoma in situ in patients with complete eradication of invasive breast cancer after neoadjuvant chemotherapy does not adversely affect patient outcome. J Clin Oncol, 2007; 125(19):2650-5). HER2 overexpression increases risk of relapse for patients at all stages of EBC. Even tumors ≤1 cm in size have been associated with a risk of relapse approaching 25% (Gonzalez-Angulo A, Litton J, Broglio K, et al. High risk of recurrence for patients with breast cancer who have human epidermal growth factor receptor 2 positive, node--negative tumors 1cm or smaller. J Clin Oncol, 2009; 27(34):5700-6). Given the high risk of relapse, the majority of patients with early stage HER2-positive breast cancer are treated with systemic therapy. The most routinely followed standard regimens for curable HER2-positive breast cancer contain two to three cytotoxic chemotherapy drugs administered in combination with Trastuzumab. With current systemic therapy approaches, a significant number of patients will still have fatal relapse of their HER2-positive breast cancer with long-term risk of relapse of 20% or higher.

Four large randomized studies that evaluated the role of Trastuzumab as adjuvant treatment of HER2-positive early stage breast cancer have been reported. In the HERCEPTIN® Adjuvant (HERA) study, patients who had completed chemotherapy were randomized to observation, or 1 year or 2 years of Trastuzumab (Piccart-Gebhart MJ, Procter M, Leyland-Jones B, et al. Trastuzumab after adjuvant chemotherapy in HER2-positive breast cancer. N Engl J Med, 2005; 353(16):1659-1672). The results for this study, including data only from the observation and 1-year duration of Trastuzumab therapy arms (Smith I. Trastuzumab following adjuvant chemotherapy in HER2-positive early breast cancer (HERA trial): disease-free and overall survival after 2 year median follow-up. *Proc ASCO,* 2006; Late Breaking Scientific Session), showed that at a median follow up of 23 months, 1 year of Trastuzumab therapy was associated with a statistically significant absolute disease-free survival (DFS) benefit of 6.3% (hazard ratio [HR]=0.64). Importantly, patients treated in the Trastuzumab arm had a 34% relative reduction in their risk of death (HR=0.66; p=0.0115). This benefit was seen in patients with both lymph node-positive and lymph node-negative disease. After median follow-up of 8 years, overall survival (OS) remained significantly better in the 1-year Trastuzumab arm compared with observation alone (HR=0.76, p-0.0005) (Gelber R, Goldhirsch A and Piccart M. HERA Trial: 2 years versus 1 year of Trastuzumab after adjuvant chemotherapy in women with HER2-positive early breast cancer at 8 years of median follow up. 2012 *ESMO Congress;* Abstract LBA6).

A combined analysis of two adjuvant treatment studies, National Surgical Adjuvant Breast and Bowel Project (NSABP) B-31 and the North Central Cancer Treatment Group (NCCTG) 9831 was conducted (Romond E, Perez E, Bryant J, et al. Trastuzumab plus adjuvant chemotherapy for operable HER2-positive breast cancer. N Engl J Med, 2005; 353(16):1673-1684). NSABP B-31 studied doxorubicin+cyclophosphamide (AC) followed by paclitaxel administered every 3 weeks (q3w) with or without 52 weeks of Trastuzumab therapy. The NCCTG 9831 compared three regimens: AC followed by weekly paclitaxel, AC followed by weekly paclitaxel followed by Trastuzumab for 52 weeks, and AC followed by a combination of weekly paclitaxel + Trastuzumab with subsequent single agent Trastuzumab for a total of 52 weeks of HER2-directed therapy. The joint analysis combined data from the control and concurrent paclitaxel + Trastuzumab arms of both studies. The authors reported an absolute benefit in 3-year DFS of 12% at 3 years. The cumulative incidence of Class III or IV congestive heart failure (CHF) or death from cardiac causes was 4.1% in the B-31 study and 2.9% in the NCCTG 9831 study in the concurrent paclitaxel and Trastuzumab arms. In addition, patients who received concurrent paclitaxel and Trastuzumab had a trend towards improvement in DFS compared with patients who received sequential paclitaxel followed by Trastuzumab (HR: 0.77; CI 0.53-1.11; p =0.02).

The fourth large study that evaluated the adjuvant use of Trastuzumab combined with chemotherapy for HER2-positive EBC was the Breast Cancer International Research Group (BCIRG) 006 study (Slamon D, Eiermann W, Robert N, et al. Adjuvant Trastuzumab in HER2-positive breast cancer. N Engl J Med, 2011; 365(14): 1273-1283). BCIRG006 was designed to determine if the introduction of Trastuzumab in early-stage HER2-positive breast cancer significantly improves clinical outcomes and if the increased cardiotoxicity observed with Trastuzumab when used with anthracyclines may be avoided by using a novel regimen of docetaxel without anthracyclines. Patients were randomly assigned to one of three treatment arms: doxorubicin and cyclophosphamide followed by docetaxel (AC-T); doxorubicin and cyclophosphamide followed by docetaxel and Trastuzumab (AC-TH); or TCH. Trastuzumab was infused weekly during chemotherapy and then q3w thereafter for a total of 52 weeks. The 5-year DFS rate for patients in the AC-T arm was 75% compared with 84% for those in the AC-TH arm (HR for comparison with AC-T, 0.65; P<0.001) and 81% in the TCH arm (HR for comparison with AC-T, 0.75; P=0.04). Similarly, OS was improved in the Trastuzumab arms compared with the AC-T arm. The 5-year OS for AC-T was 87% compared to 92% for AC-TH (HR, 0.63; P<0.001) and 91% for TCH (HR, 0.77; P = 0.04). While the study was not powered to detect equivalence between the two Trastuzumab-based regimens, there was no significant difference in the rate of DFS or OS between TCH and AC-TH. Importantly, there were fewer Grade 3 or 4 CHF events in the TCH arm when compared with the anthracycline/Trastuzumab arm (4 vs. 21 respectively, p<0.001). Moreover, subclinical loss of mean left ventricular ejection fraction (LVEF) >10% was seen in 18.6% of patients in the AC-TH arm compared to 9.4% of patients in the TCH arm (P<0.001). Although many patients who received Trastuzumab in BCIRG had recovery of cardiac function, of the 18.6% of patients receiving AC-TH that had a relative reduction in LVEF of >10%, the decrease was persistent in many, lasting >4 years in 33% of these patients. Mean change of LVEF from baseline at month 42 was -3.5 for AC-TH in contrast to 0.2 for the TCH treatment group (data on file). The TCH regimen carries similar efficacy with fewer acute toxic effects and lower risks of cardiotoxicity and leukemia in the adjuvant setting than anthracycline-based regimens.

### Neoadjuvant Therapy for HER2-Positive Breast Cancer

Multiple studies have evaluated Trastuzumab combined with different chemotherapeutic agents in the preoperative setting (Burstein H, Harris L, Gelman R, et al. Preoperative therapy with Trastuzumab and paclitaxel followed by sequential adjuvant doxorubicin/cyclophosphamide for HER2 overexpressing stage II or III breast cancer: a pilot study. J Clin Oncol, 2003; 21(1):46-53; Buzdar A, Ibrahim N, Francis D. Significantly higher pathologic complete remission rate after neoadjuvant therapy with Trastuzumab, paclitaxel, and epirubicin chemotherapy: results of a randomized trial in human epidermal growth factor receptor 2-positive operable breast cancer. J Clin Oncol. 2005; 23(16):3676-3685; Gianni L, Eiermann W, Semiglazov V, et al. Neoadjuvant chemotherapy with Trastuzumab followed by adjuvant Trastuzumab versus neoadjuvant chemotherapy alone, in patients with HER2-positive locally advanced breast cancer (the NOAH trial): a randomised controlled superiority trial with a parallel HER2-negative cohort. Lancet, 2010; 375(9712):377-384; Untch M, Rezai M, Loibl S, et al. Neoadjuvant treatment with Trastuzumab in HER2-positive breast cancer: results from the GeparQuattro study. J Clin Oncol, 2010; 28(12): 2024-2031; Untch M, Fasching PA, Konecny GE, et al. Pathologic complete response after neoadjuvant chemotherapy plus Trastuzumab predicts favorable survival in human epidermal growth factor receptor-2-overexpressing breast cancer : results from the TECHNO trial of the AGO and GBG study groups. J Clin Oncol. 2011; 29(25):3351-7; Dent S, Oyan B, Honig A et al. HER2-targeted therapy in breast cancer: A systematic review of neoadjuvant trials. Cancer Treat Rev. 2013 Oct;39(6):622-31). One of the first of these trials was a pilot study that evaluated the safety and efficacy of paclitaxel combined with Trastuzumab. This regimen yielded a pCR rate of 18% and clinical response rate of 85% (Burstein et al. 2003, supra). A subsequent trial reported that patients who received neoadjuvant anthracycline-based polychemotherapy+Trastuzumab achieved a pCR rate of 65%, compared to only 26% of patients who received chemotherapy alone (p=0.016) (Buzdar et al. 2005 supra).

One of the largest neoadjuvant trials in the HER2-positive population was a Phase III, randomized study comparing the safety and efficacy of a sequential neoadjuvant regimen, including doxorubicin, paclitaxel, and cyclophosphamide, methotrexate, and 5-fluorouracil (CMF), with or without Trastuzumab in 333 patients with HER2-positive locally advanced breast cancer (NOAH trial). A parallel observational control group of HER2-negative patients received the same chemotherapy regimen (Gianni et al. 2010, supra). The addition of Trastuzumab to neoadjuvant chemotherapy and continuation of Trastuzumab therapy in the adjuvant setting for a total of one year resulted in a clinically relevant and statistically significant improvement in event-free survival (EFS) and OS in previously untreated patients with locally advanced HER2-positive breast cancer. These data are supported by results of the secondary efficacy parameters.

In the NOAH study, the addition of Trastuzumab to a full chemotherapy regimen was associated with an increase in bpCR of 17.6 % (from 26.7% to 44.3%). The increase in bpCR translated into an improved EFS. Neoadjuvant therapy with Trastuzumab followed by adjuvant Trastuzumab was approved in the EU based upon results of this trial.

Other Trastuzumab-based preoperative treatment regimens have been studied for breast cancer demonstrating activity in HER2-positive disease both in combination with chemotherapy as well as with other HER2-directed therapies. The administration of TCH in the neoadjuvant setting resulted in pCR rates in the breast and lymphatics between 38.5-43.7% (Guiu S, Liegard M, Favier L et al. Long-term follow-up of HER2-overexpressing Stage II or III breast cancer treated by anthracycline-free neoadjuvant chemotherapy. Ann Oncol. 2011; 22(2):321-8; Bayraktar S, Gonzalez-Angulo A, Lei X et al. Efficacy of neoadjuvant therapy with Trastuzumab concurrent with anthracycline- and nonanthracycline-based regimens for HER2-positive breast cancer. Cancer. 2012; 118(9):2385-2393).

### Adjuvant Therapy for HER2 Positive Breast Cancer

Adjuvant therapy, in the broadest sense, is treatment given in addition to the primary therapy to kill any cancer cells that may have spread, even if the spread cannot be detected by radiologic or laboratory tests.

Publications or seminars related to adjuvant therapy include: Paik et al., J. Natl. Cancer Inst., 92(24):1991-1998 (2000); Paik et al., J. Natl. Cancer Inst., 94:852-854 (2002); Paik et al. Successful quality assurance program for HER2 testing in the NSABP Trial for Herceptin. San Antonio Breast Cancer Symposium, 2002; Roche PC et al., J. Natl. Cancer Inst., 94(11):855-7 (2002); Albain et al., Proceedings of the American Society of Clinical Oncology Thirty-Eighth Annual Meeting, May 18-21 2002, Orlando, FL, Abstract 143; The ATAC (Arimidex, Tamoxifen Alone or in Combination) Trialists' Group, Lancet, 359:2131-39 (2002); Geyer et al., 26th Annual San Antonio Breast Cancer Symposium (SABCS), December 2003, Abstract 12; Perez et al., Proc. ASCO, 2005, Abstract 556.

U.S. Patent Publication No. 2004/0014694 (published January 22, 2004) describes a method of adjuvant therapy for the treatment of early breast cancer, comprising administration of docetaxel, doxorubicin and cyclophosphamide. U.S. Patent Publication No. 2006/0275305 describes methods of adjuvant therapy using Trastuzumab and Trastuzumab-drug conjugates.

Currently utilized HER2-directed therapy for EBC leaves a significant number of patients at risk for relapse and death from their disease. There is a great need for further treatment options, which improve outcome, preferably including significant improvements in the ability to eradicate invasive cancer in the breast and the lymph nodes.

### SUMMARY OF THE INVENTION

The invention relates generally to methods of treating breast cancer patients with the antibody-drug conjugate, Trastuzumab-MCC-DMl (T-DM1) and Pertuzumab.

In one aspect, the invention concerns a method for the treatment of breast cancer, comprising
(i) subjecting a patient with HER2-positive, operable, locally advanced or inflammatory breast cancer to neoadjuvant treatment with a combination of T-DM1 and Pertuzumab, in the absence of chemotherapy,
(ii) removing said breast cancer by definitive surgery; and
(iii) subjecting said patient to adjuvant treatment with a combination of T-DM1 and Pertuzumab, in the absence of chemotherapy.

In one embodiment, the patient is subjected to adjuvant treatment with a combination of T-DM1 and Pertuzumab, in the absence of chemotherapy that comprises a taxane.

In another embodiment, the patient is subjected to adjuvant treatment with a combination of T-DM1 and Pertuzumab, in the absence of concurrent chemotherapy, prior to and/or following definitive surgery.

In yet another embodiment, the adjuvant treatment comprises chemotherapy prior to and/or following treatment with T-DM1 and Pertuzumab.

In a further embodiment, the chemotherapy prior to and/or following treatment with T-DM1 and Pertuzumab does not comprise a taxane.

In a still further embodiment, the chemotherapy that is administered comprises anthracycline-based chemotherapy.

In another embodiment, the chemotherapy that is administered further comprises Trastuzumab.

In all embodiments, anthacycline-based therapy, if present, may, for example, comprise one or more of FAC (5-fluoroacil, doxorubicin, cyclophosphamide), FEC (5-fluorouracil, epirubicin and cyclophosphamide) or AC (doxorubicin, cyclophosphamide).9.

In one embodiment, the breast cancer is >2 cm in diameter.

In another embodiment, definitive surgery is performed at least 14 days following the completion of neoadjuvant therapy.

In yet another embodiment, definitive surgery is performed no later than 9 weeks following the completion of neoadjuvant therapy.

In a further embodiment, the neoadjuvant and adjuvant treatment protocols each comprise infusion of T-DM1 at a dose of 3.6 mg/kg every 3 weeks and infusion of Pertuzumab at a loading dose of 840mg and at a dose of 420mg every 3 weeks thereafter.

In all embodiments, T-DM1 and Pertuzumab may be administered concurrently, may be co-administered, or may be administered consecutively in either order. In one particular embodiment, the administration follows the schedule set forth in Table 5.

In a further embodiment, the treatment increases one or more of complete response (CR), EFS (event-free survival), DFS (disease-free survival), IDFS (invasive diseas-free survival), and OS (overall survival).

In a still further embodiment, the treatment increases time to disease progression.

In one embodiment, the neoadjuvant treatment consists essentially of administration of T-DM1 and Pertuzumab.

In another embodiment, the neoadjuvant treatment consists of administration of T-DM1 and Pertuzumab.

In a further embodiment, the adjuvant treatment consists essentially of administration of T-DM1 and Pertuzumab.

In a still further embodiment, the adjuvant treatment consists of administration of T-DM1 and Pertuzumab.

### BRIEF DESCRIPTION OF THE DRAWINGS

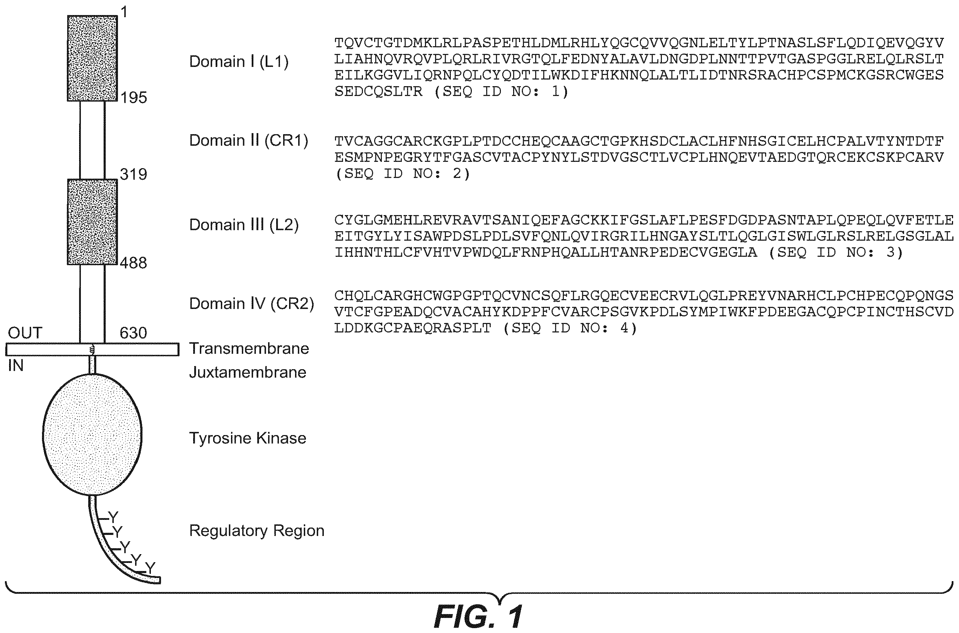

FIG. 1 provides a schematic of the HER2 protein structure, and amino acid sequences for Domains I-IV (SEQ ID Nos. 1-4, respectively) of the extracellular domain thereof.

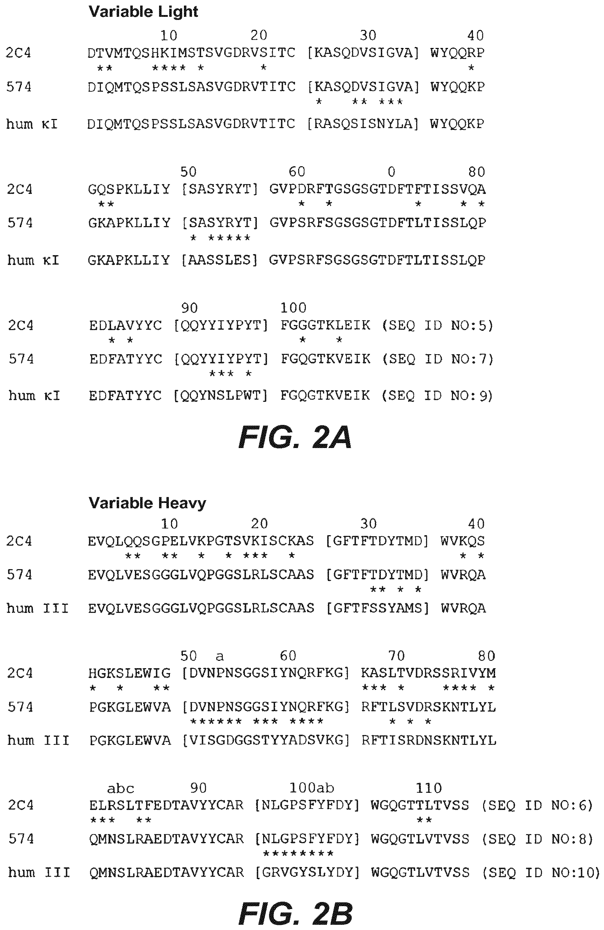

FIGS. 2A and 2B depict alignments of the amino acid sequences of the variable light (V_{L}) (FIG. 2A) and variable heavy (V_{H}) (FIG. 2B) domains of murine monoclonal antibody 2C4 (SEQ ID Nos. 5 and 6, respectively); V_{L} and V_{H} domains of variant 574/Pertuzumab (SEQ ID Nos. 7 and 8, respectively), and human V_{L} and V_{H} consensus frameworks (hum id, light kappa subgroup I; humIII, heavy subgroup III) (SEQ ID Nos. 9 and 10, respectively). Asterisks identify differences between variable domains of Pertuzumab and murine monoclonal antibody 2C4 or between variable domains of Pertuzumab and the human framework. Complementarity Determining Regions (CDRs) are in brackets.

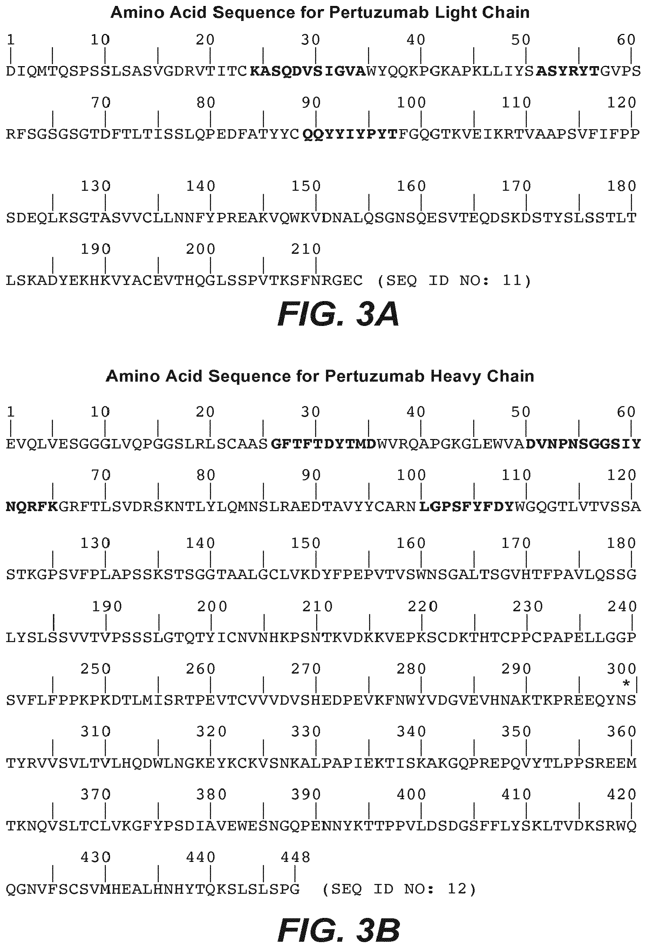

FIGS. 3A and 3B show the amino acid sequences of Pertuzumab light chain (FIG. 3A; SEQ ID NO. 11) and heavy chain (FIG. 3B; SEQ ID No. 12). CDRs are shown in bold. Calculated molecular mass of the light chain and heavy chain are 23,526.22 Da and 49,216.56 Da (cysteines in reduced form). The carbohydrate moiety is attached to Asn 299 of the heavy chain.
FIGS. 4A and 4B show the amino acid sequences of Trastuzumab light chain (FIG. 4A; SEQ ID NO. 13) and heavy chain (FIG. 4B; SEQ ID NO. 14), respectively. Boundaries of the variable light and variable heavy domains are indicated by arrows.

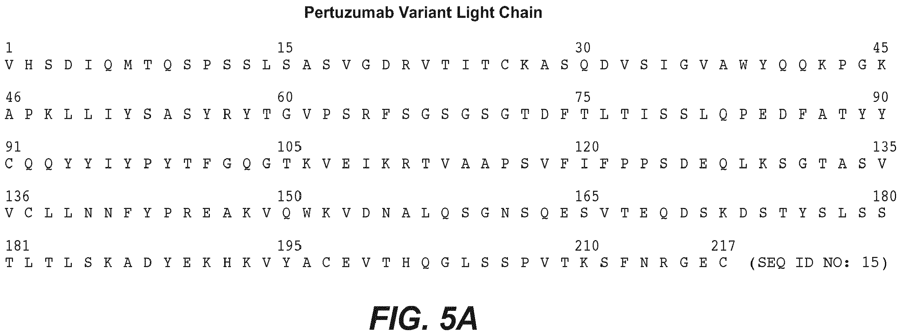

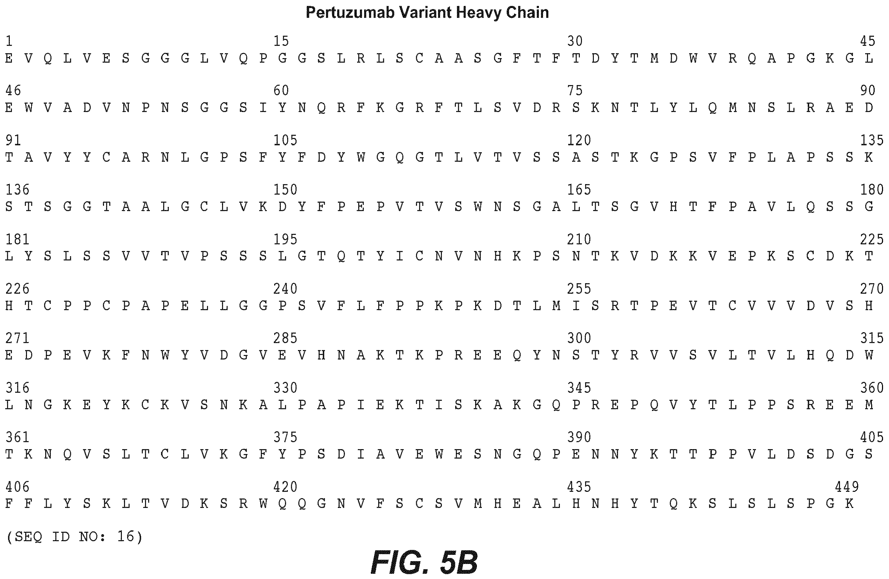

FIGS. 5A and 5B depict a variant Pertuzumab light chain sequence (FIG. 5A; SEQ ID NO. 15) and a variant Pertuzumab heavy chain sequence (FIG. 5B; SEQ ID NO. 16), respectively.
FIG. 6 depicts the schema of KRISTINE clinical trial described in Example 1.

### DETAILED DESCRIPTION OF EXEMPLARY EMBODIMENTS

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described.

All references cited throughout the disclosure are expressly incorporated by reference herein in their entirety. In the event that one or more of the incorporated literature, patents, and similar materials differs from or contradicts this application, including but not limited to defined terms, term usage, described techniques, or the like, this application controls.

### DEFINITIONS

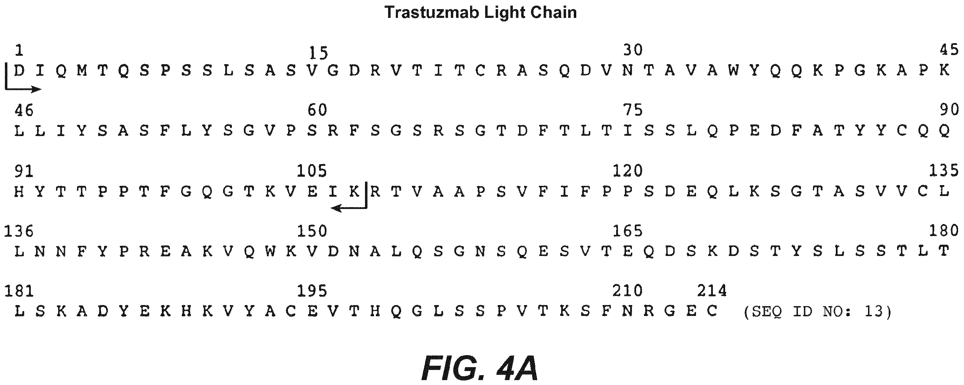

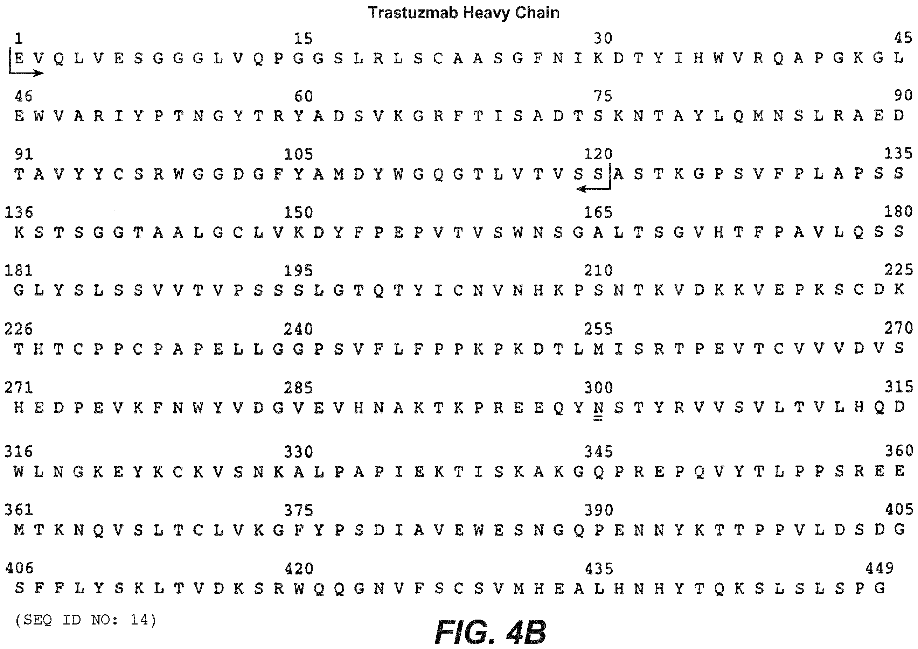

The words "comprise," "comprising," "include," "including," and "includes" when used in this specification and claims are intended to specify the presence of stated features, integers, components, or steps, but they do not preclude the presence or addition of one or more other features, integers, components, steps, or groups thereof.
The terms "treat" and "treatment" refer to both therapeutic treatment and prophylactic or preventative measures, wherein the object is to prevent or slow down (lessen) an undesired physiological change or disorder, such as the growth, development or spread of a hyperproliferative condition, such as cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.
The terms "cancer" and "cancerous" refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.
The term "early stage breast cancer (EBC)" or "early breast cancer" is used herein to refer to breast cancer that has not spread beyond the breast or the axillary lympho nodes. This includes ductal carcinoma in situ and stage I, stage IIA, stage IIB, and stage IIIA breast cancers.
Reference to a tumor or cancer as a "Stage 0," "Stage I," "Stage II," "Stage III," or "Stage IV", and various sub-stages within this classification, indicates classification of the tumor or cancer using the Overall Stage Grouping or Roman Numeral Staging methods known in the art. Although the actual stage of the cancer is dependent on the type of cancer, in general, a Stage 0 cancer is an in situ lesion, a Stage I cancer is small localized tumor, a Stage II and III cancer is a local advanced tumor which exhibits involvement of the local lymph nodes, and a Stage IV cancer represents metastatic cancer. The specific stages for each type of tumor is known to the skilled clinician.
The term "metastatic breast cancer" means the state of breast cancer where the cancer cells are transmitted from the original site to one or more sites elsewhere in the body, by the blood vessels or lymphatics, to form one or more secondary tumors in one or more organs besides the breast.
An "advanced" cancer is one which has spread outside the site or organ of origin, either by local invasion or metastasis. Accordingly, the term "advanced" cancer includes both locally advanced and metastatic disease.
A "refractory" cancer is one which progresses even though an anti-tumor agent, such as a chemotherapy, is being administered to the cancer patient. An example of a refractory cancer is one which is platinum refractory.
A "recurrent" cancer is one which has regrown, either at the initial site or at a distant site, after a response to initial therapy, such as surgery.
A "locally recurrent" cancer is cancer that returns after treatment in the same place as a previously treated cancer.
An"operable" or "resectable" cancer is cancer which is confined to the primary organ and suitable for surgery (resection).
A "non-resectable" or "unresectable" cancer is not able to be removed (resected) by surgery.
A "HER2-positive" cancer comprises cancer cells which have higher than normal levels of HER2. Examples of HER2-positive cancer include HER2-positive breast cancer and HER2-positive gastric cancer. Optionally, HER2-positive cancer has an immunohistochemistry (IHC) score of 2+ or 3+ and/or an *in situ* hybridization (ISH) amplification ratio ≥2.0.
Herein, a "patient" or "subject" is a human patient. The patient may be a "cancer patient," *i.e.* one who is suffering or at risk for suffering from one or more symptoms of cancer, in particular gastric or breast cancer.
A "patient population" refers to a group of cancer patients. Such populations can be used to demonstrate statistically significant efficacy and/or safety of a drug, such as Pertuzumab.
A "relapsed" patient is one who has signs or symptoms of cancer after remission. Optionally, the patient has relapsed after adjuvant or neoadjuvant therapy.
A cancer or biological sample which "displays HER expression, amplification, or activation" is one which, in a diagnostic test, expresses (including overexpresses) a HER receptor, has amplified HER gene, and/or otherwise demonstrates activation or phosphorylation of a HER receptor.
"Neoadjuvant therapy" or "preoperative therapy" herein refers to therapy given prior to surgery. The goal of neoadjuvant therapy is to provide immediate systemic treatment, potentially eradicating micrometastases that would otherwise proliferate if the standard sequence of surgery followed by systemic therapy were followed. Neoadjuvant therapy may also help to reduce tumor size thereby allowing complete resection of initially unresectable tumors or preserving portions of the organ and its functions. Furthermore, neoadjuvant therapy permits an in vivo assessment of drug efficacy, which may guide the choice of subsequent treatments.
"Adjuvant therapy" herein refers to therapy given after definitive surgery, where no evidence of residual disease can be detected, so as to reduce the risk of disease recurrence. The goal of adjuvant therapy is to prevent recurrence of the cancer, and therefore to reduce the chance of cancer-related death. Adjuvant therapy herein specifically excludes neoadjuvant therapy.
"Definitive surgery" is used as that term is used within the medical community. Definitive surgery includes, for example, procedures, surgical or otherwise, that result in removal or resection of the tumor, including those that result in the removal or resection of all grossly visible tumor. Definitive surgery includes, for example, complete or curative resection or complete gross resection of the tumor. Definitive surgery includes procedures that occur in one or more stages, and includes, for example, multi-stage surgical procedures where one or more surgical or other procedures are performed prior to resection of the tumor. Definitive surgery includes procedures to remove or resect the tumor including involved organs, parts of organs and tissues, as well as surrounding organs, such as lymph nodes, parts of organs, or tissues. Removal may be incomplete such that tumor cells might remain even though undetected.
"Survival" refers to the patient remaining alive, and includes disease free survival (DFS), progression free survival (PFS) and overall survival (OS). Survival can be estimated by the Kaplan-Meier method, and any differences in survival are computed using the stratified log-rank test.
"Progression-Free Survival" (PFS) is the time from the first day of treatment to documented disease progression (including isolated CNS progression) or death from any cause on study, whichever occurs first.
"Disease free survival (DFS)" refers to the patient remaining alive, without return of the cancer, for a defined period of time such as about 1 year, about 2 years, about 3 years, about 4 years, about 5 years, about 10 years, etc., from initiation of treatment or from initial diagnosis. In one aspect of the invention, DFS is analyzed according to the intent-to-treat principle, i.e., patients are evaluated on the basis of their assigned therapy. The events used in the analysis of DFS can include local, regional and distant recurrence of cancer, occurrence of secondary cancer, and death from any cause in patients without a prior event (e.g, breast cancer recurrence or second primary cancer).
"Overall survival" refers to the patient remaining alive for a defined period of time, such as about 1 year, about 2 years, about 3 years, about 4 years, about 5 years, about 10 years, etc., from initiation of treatment or from initial diagnosis. In the studies underlying the invention the event used for survival analysis was death from any cause.
By "extending survival" is meant increasing DFS and/or OS in a treated patient relative to an untreated patient, or relative to a control treatment protocol. Survival is monitored for at least about six months, or at least about 1 year, or at least about 2 years, or at least about 3 years, or at least about 4 years, or at least about 5 years, or at least about 10 years, etc., following the initiation of treatment or following the initial diagnosis.
"Hazard ratio" in survival analysis is a summary of the difference between two survival curves, representing the reduction in the risk of death on treatment compared to control, over a period of follow-up. Hazard ratio is a statistical definition for rates of events. For the purpose of the invention, hazard ratio is defined as representing the probability of an event in the experimental arm divided by the probability of an event in the control arm at any specific point in time.
By "monotherapy" is meant a therapeutic regimen that includes only a single therapeutic agent for the treatment of the cancer or tumor during the course of the treatment period.
By "maintenance therapy" is meant a therapeutic regimen that is given to reduce the likelihood of disease recurrence or progression. Maintenance therapy can be provided for any length of time, including extended time periods up to the life-span of the subject. Maintenance therapy can be provided after initial therapy or in conjunction with initial or additional therapies. Dosages used for maintenance therapy can vary and can include diminished dosages as compared to dosages used for other types of therapy.
As defined herein, the terms "Trastuzumab", "HERCEPTIN®" and "huMAb4D5-8" are used interchangeably. Such antibody preferably comprises the light and heavy chain amino acid sequences shown in FIGS. 4A (SEQ ID NO: 13) and FIG. 4B (SEQ ID NO. 14), respectively.
The "epitope 4D5" or "4D5 epitope" or "4D5" is the region in the extracellular domain of HER2 to which the antibody 4D5 (ATCC CRL 10463) and Trastuzumab bind. This epitope is close to the transmembrane domain of HER2, and within Domain IV of HER2. To screen for antibodies which bind to the 4D5 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed to assess whether the antibody binds to the 4D5 epitope of HER2 (e.g. any one or more residues in the region from about residue 529 to about residue 625, inclusive, of HER2).
The "epitope 2C4" or "2C4 epitope" is the region in the extracellular domain of HER2 to which the antibody 2C4 binds. In order to screen for antibodies which bind to the 2C4 epitope, a routine cross-blocking assay such as that described in Antibodies, A Laboratory Manual, Cold Spring Harbor Laboratory, Ed Harlow and David Lane (1988), can be performed. Alternatively, epitope mapping can be performed to assess whether the antibody binds to the 2C4 epitope of HER2. Epitope 2C4 comprises residues from domain II in the extracellular domain of HER2. The 2C4 antibody and Pertuzumab bind to the extracellular domain of HER2 at the junction of domains I, II and III (Franklin et al. Cancer Cell 5:317-328 (2004)).
For the purposes herein, "Pertuzumab", "PERJETA®" and "rhuMAb 2C4", are used interchangeably. Such antibody preferably comprises the light and heavy chain amino acid sequences in SEQ ID NOs: 7 and 8, respectively. Where Pertuzumab is an intact antibody, it preferably comprises an IgG1 antibody; in one embodiment comprising the light chain amino acid sequence in SEQ ID NO: 11 or 15, and heavy chain amino acid sequence in SEQ ID NO: 12 or 16. The antibody is optionally produced by recombinant Chinese Hamster Ovary (CHO) cells.
As defined herein, the terms "T-DM1," "Trastuzumab-MCC-DMl," "ado-Trastuzumab emtansine," "Trastuzumab emtansine," and "KADCYLA®" are used interchangeably, and refer to Trastuzumab linked through the linker moiety MCC to the maytansinoid drug moiety DM1, including all mixtures of variously loaded and attached antibody-drug conjugates where 1, 2, 3, 4, 5, 6, 7, and 8 drug moieties are covalently attached to the antibody Trastuzumab (US 7097840; US 2005/0276812; US 2005/0166993).
Herein, an "anti-tumor agent" refers to a drug used to treat cancer. Non-limiting examples of anti-tumor agents herein include chemotherapy agents, HER dimerization inhibitors, HER antibodies, antibodies directed against tumor associated antigens, anti-hormonal compounds, cytokines, EGFR-targeted drugs, anti-angiogenic agents, tyrosine kinase inhibitors, growth inhibitory agents and antibodies, cytotoxic agents, antibodies that induce apoptosis, COX inhibitors, farnesyl transferase inhibitors, antibodies that binds oncofetal protein CA 125, HER2 vaccines, Raf or ras inhibitors, liposomal doxorubicin, topotecan, taxane, dual tyrosine kinase inhibitors, TLK286, EMD-7200, Pertuzumab, Trastuzumab, erlotinib, and bevacizumab.
A "chemotherapy" is use of a chemotherapeutic agent useful in the treatment of cancer.
A "chemotherapeutic agent" is a chemical compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors. Examples of chemotherapeutic agents include: erlotinib (TARCEVA®, Genentech/OSI Pharm.), docetaxel (TAXOTERE®, Sanofi-Aventis), 5-FU (fluorouracil, 5-fluorouracil, CAS No. 51-21-8), gemcitabine (GEMZAR®, Lilly), PD-0325901 (CAS No. 391210-10-9, Pfizer), cisplatin (cis-diamine,dichloroplatinum(II), CAS No. 15663-27-1), carboplatin (CAS No. 41575-94-4), paclitaxel (TAXOL®, Bristol-Myers Squibb Oncology, Princeton, N.J.), temozolomide (4-methyl-5-oxo- 2,3,4,6,8-pentazabicyclo [4.3.0] nona-2,7,9-triene- 9-carboxamide, CAS No. 85622-93-1, TEMODAR®, TEMODAL®, Schering Plough), tamoxifen ((Z)-2-[4-(1,2-diphenylbut-1-enyl)phenoxy]-*N,N*-dimethyl-ethanamine, NOLVADEX®, ISTUBAL®, VALODEX®), and doxorubicin (ADRIAMYCIN®), Akti-1/2, HPPD, and rapamycin.
More examples of chemotherapeutic agents include: oxaliplatin (ELOXATIN®, Sanofi), bortezomib (VELCADE®, Millennium Pharm.), sutent (SUNITINIB®, SU11248, Pfizer), letrozole (FEMARA®, Novartis), imatinib mesylate (GLEEVEC®, Novartis), XL-518 (MEK inhibitor, Exelixis, WO 2007/044515), ARRY-886 (Mek inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX®, AstraZeneca), leucovorin (folinic acid), rapamycin (sirolimus, RAPAMUNE®, Wyeth), lapatinib (TYKERB®, GSK572016, Glaxo Smith Kline), lonafarnib (SARASAR™, SCH 66336, Schering Plough), sorafenib (NEXAVAR®, BAY43-9006, Bayer Labs), gefitinib (IRESSA®, AstraZeneca), irinotecan (CAMPTOSAR®, CPT-11, Pfizer), tipifarnib (ZARNESTRA™, Johnson & Johnson), ABRAXANE™ (Cremophor-free), albumin-engineered nanoparticle formulations of paclitaxel (American Pharmaceutical Partners, Schaumberg, II), vandetanib (rINN, ZD6474, ZACTIMA®, AstraZeneca), chloranmbucil, AG1478, AG1571 (SU 5271; Sugen), temsirolimus (TORISEL®, Wyeth), pazopanib (GlaxoSmithKline), canfosfamide (TELCYTA®, Telik), thiotepa and cyclosphosphamide (CYTOXAN®, NEOSAR®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, calicheamicin gamma1I, calicheamicin omegaI1 (Angew Chem. Intl. Ed. Engl. (1994) 33:183-186); dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (T-2 toxin, verracurin A, roridin A and anguidine); urethan; vindesine; dacarbazine; mannomustine; mitobronitol; mitolactol; pipobroman; gacytosine; arabinoside (Ara-C); cyclophosphamide; thiotepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE®); novantrone; teniposide; edatrexate; daunomycin; aminopterin; capecitabine (XELODA®, Roche); ibandronate; CPT-11; topoisomerase inhibitor RFS 2000; difluoromethylornithine (DMFO); retinoids such as retinoic acid; and pharmaceutically acceptable salts, acids and derivatives of any of the above.
The term "effective amount" refers to an amount of a drug effective to treat cancer in the patient. The effective amount of the drug may reduce the number of cancer cells; reduce the tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. The effective amount may extend progression free survival *(e.g.* as measured by Response Evaluation Criteria for Solid Tumors, RECIST, or CA-125 changes), result in an objective response (including a partial response, PR, or complete response, CR), increase overall survival time, and/or improve one or more symptoms of cancer (*e.g.* as assessed by FOSI). The term "effective amount" specifically includes an amount suitable for achieving any of the primary or secondary endpoints of the clinical trial described in Example 1.
A "taxane" is a chemotherapy which inhibits mitosis and interferes with microtubules. Examples of taxanes include paclitaxel (TAXOL®; Bristol-Myers Squibb Oncology, Princeton, N.J.); cremophor-free, albumin-engineered nanoparticle formulation of paclitaxel or *nab*-paclitaxel (ABRAXANE™; American Pharmaceutical Partners, Schaumberg, Illinois); and docetaxel (TAXOTERE®; Rhône-Poulenc Rorer, Antony, France).
An "anthacycline" is a type of antibiotic that comes from the fungus Streptococcus peucetius, examples include: daunorubicin, doxorubicin, and epirubicin, etc.
"Anthracycline-based chemotherapy" refers to a chemotherapy regimen that consists of or include one or more anthracycline. Examples include 5-FU, epirubicin, and cyclophosphamide (FEC); 5-FU, doxorubicin, and cyclophosphamide (FAC); doxorubicin and cyclophosphamide (AC); epirubicin and cyclophosphamide (EC); etc.
For the purposes herein, "carboplatin-based chemotherapy" refers to a chemotherapy regimen that consists of or includes one or more carboplatins. An example is TCH (docetaxel/TAXOL®, carboplatin, and Trastuzumab/HERCEPTIN®).
An "aromatase inhibitor" inhibits the enzyme aromatase, which regulates estrogen production in the adrenal glands. Examples of aromatase inhibitors include: 4(5)-imidazoles, aminoglutethimide, MEGASE® megestrol acetate, AROMASIN® exemestane, formestane, fadrozole, RIVISOR® vorozole, FEMARA® letrozole, and ARIMIDEX® anastrozole. In one embodiment, the aromatase inhibitor herein is letrozole or anastrozole.
An "antimetabolite chemotherapy" is use of an agent which is structurally similar to a metabolite, but can not be used by the body in a productive manner. Many antimetabolite chemotherapeutic agents interfere with the production of the nucleic acids, RNA and DNA. Examples of antimetabolite chemotherapeutic agents include gemcitabine (GEMZAR®), 5-fluorouracil (5-FU), capecitabine (XELODA™), 6-mercaptopurine, methotrexate, 6-thioguanine, pemetrexed, raltitrexed, arabinosylcytosine ARA-C cytarabine (CYTOSAR-U®), dacarbazine (DTIC-DOME®), azocytosine, deoxycytosine, pyridmidene, fludarabine (FLUDARA®), cladrabine, 2-deoxy-D-glucose etc.
By "chemotherapy-resistant" cancer is meant that the cancer patient has progressed while receiving a chemotherapy regimen (*i.e.* the patient is "chemotherapy refractory"), or the patient has progressed within 12 months (for instance, within 6 months) after completing a chemotherapy regimen.
The term "platin" is used herein to refer to platinum based chemotherapy, including, without limitation, cisplatin, carboplatin, and oxaliplatin.
The term "fluoropyrimidine" is used herein to refer to an antimetabolite chemotherapy, including, without limitation, capecitabine, floxuridine, and fluorouracil (5-FU).
A "fixed " or "flat" dose of a therapeutic agent herein refers to a dose that is administered to a human patient without regard for the weight (WT) or body surface area (BSA) of the patient. The fixed or flat dose is therefore not provided as a mg/kg dose or a mg/m² dose, but rather as an absolute amount of the therapeutic agent.
A "loading" dose herein generally comprises an initial dose of a therapeutic agent administered to a patient, and is followed by one or more maintenance dose(s) thereof. Generally, a single loading dose is administered, but multiple loading doses are contemplated herein. Usually, the amount of loading dose(s) administered exceeds the amount of the maintenance dose(s) administered and/or the loading dose(s) are administered more frequently than the maintenance dose(s), so as to achieve the desired steady-state concentration of the therapeutic agent earlier than can be achieved with the maintenance dose(s).
A "maintenance" dose herein refers to one or more doses of a therapeutic agent administered to the patient over a treatment period. Usually, the maintenance doses are administered at spaced treatment intervals, such as approximately every week, approximately every 2 weeks, approximately every 3 weeks, or approximately every 4 weeks, preferably every 3 weeks.
"Infusion" or "infusing" refers to the introduction of a drug-containing solution into the body through a vein for therapeutic purposes. Generally, this is achieved via an intravenous (IV) bag.
An "intravenous bag" or "IV bag" is a bag that can hold a solution which can be administered via the vein of a patient. In one embodiment, the solution is a saline solution (e.g. about 0.9% or about 0.45% NaCl). Optionally, the IV bag is formed from polyolefin or polyvinal chloride.
By "co-administering" is meant intravenously administering two (or more) drugs during the same administration, rather than sequential infusions of the two or more drugs. Generally, this will involve combining the two (or more) drugs into the same IV bag prior to co-administration thereof.
A drug that is administered "concurrently" with one or more other drugs is administered during the same treatment cycle, on the same day of treatment as the one or more other drugs, and, optionally, at the same time as the one or more other drugs. For instance, for cancer therapies given every 3 weeks, the concurrently administered drugs are each administered on day-1 of a 3-week cycle.
"Cardiac toxicity" refers to any toxic side effect that affects the heart and that results from administration of a drug or drug combination. Cardiac toxicity can be evaluated based on any one or more of: incidence of symptomatic left ventricular systolic dysfunction (LVSD) or congestive heart failure (CHF), or decrease in left ventricular ejection fraction (LVEF).
The phrase "without increasing cardiac toxicity" for a drug combination including Pertuzumab refers to an incidence of cardiac toxicity that is equal or less than that observed in patients treated with drugs other than Pertuzumab in the drug combination (e.g. equal or less than that resulting from administration of Trastuzumab and the chemotherapy, e.g. docetaxel).
A "vial" is a container suitable for holding a liquid or lyophilized preparation. In one embodiment, the vial is a single-use vial, e.g. a 20-cc single-use vial with a stopper.
The term "package insert" is used to refer to instructions customarily included in commercial packages of therapeutic products, that contain information about the indications, usage, dosage, administration, contraindications and/or warnings concerning the use of such therapeutic products.
An "adverse event" is any unfavorable and unintended sign, symptom, or disease temporally associated with the use of an investigational (medicinal) product or other protocol-imposed intervention, regardless of attribution; and includes: AEs not previously observed in the patient that emerge during the protocol-specified AE reporting period, including signs or symptoms associated with breast cancer that were not present before the AE reporting period; complications that occur as a result of protocol-mandated interventions (e.g., invasive procedures such as biopsies); if applicable, AEs that occur before assignment of study treatment associated with medication washout, no treatment run-in, or other protocol-mandated intervention; Preexisting medical conditions (other than the condition being studied) judged by the investigator to have worsened in severity or frequency or changed in character during the protocol-specified AE reporting period
An adverse event is classified as a "Serious Adverse Events" (SAE) if it meets the following criteria: results in death (i.e., the AE actually causes or leads to death); life threatening (i.e., the AE, in the view of the investigator, places the patient at immediate risk of death, but not including an AE that, had it occurred in a more severe form, might have caused death); requires or prolongs inpatient hospitalization; results in persistent or significant disability/incapacity (i.e., the AE results in substantial disruption of the patient's ability to conduct normal life functions); results in a congenital anomaly/birth defect in a neonate/infant born to a mother exposed to the investigational product; or is considered a significant medical event by the investigator based on medical judgment (e.g., may jeopardize the patient or may require medical/surgical intervention to prevent one of the outcomes listed above). All AEs that do not meet any of the criteria for serious are regarded as non-serious AEs. The terms "severe" and "serious" are not synonymous. Severity (or intensity) refers to the grade of a specific AE, e.g., mild (Grade 1), moderate (Grade 2), or severe (Grade 3) myocardial infarction (see Section 5.2.2). "Serious" is a regulatory definition (see previous definition) and is based on patient or event outcome or action criteria usually associated with events that pose a threat to a patient's life or functioning. Seriousness (not severity) serves as the guide for defining regulatory reporting obligations from the Sponsor to applicable regulatory authorities. Severity and seriousness should be independently assessed when recording AEs and SAEs on the eCRF

### DETAILED DESCRIPTION

### Trastuzumab-MCC-DMl (T-DM1)

The present invention includes therapeutic treatments with Trastuzumab-MCC-DM1 (T-DM1), an antibody-drug conjugate (CAS Reg. No. 139504-50-0), which has the structure: 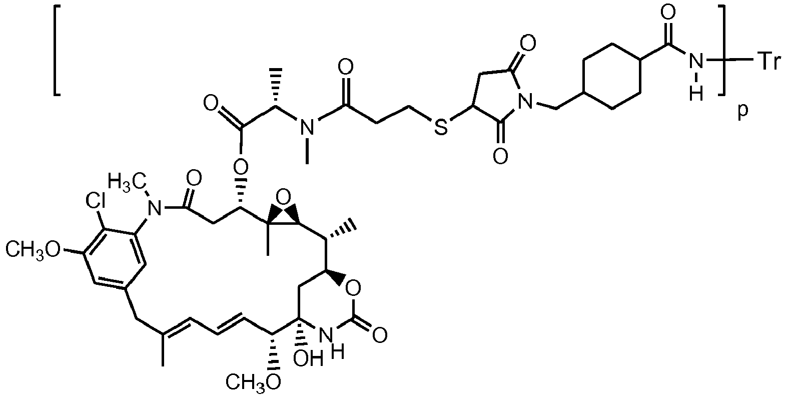 where Tr is Trastuzumab linked through linker moiety MCC to the maytansinoid drug moiety DM1 (US 5208020; US 6441163). The drug to antibody ratio or drug loading is represented by p in the above structure of Trastuzumab-MCC-DMl, and ranges in integer values from 1 to about 8. Trastuzumab-MCC-DMl includes all mixtures of variously loaded and attached antibody-drug conjugates where 1, 2, 3, 4, 5, 6, 7, and 8 drug moieties are covalently attached to the antibody Trastuzumab (US 7097840; US 2005/0276812; US 2005/0166993).

Trastuzumab can be produced by a mammalian cell (Chinese Hamster Ovary, CHO) suspension culture. The HER2 (or c-erbB2) proto-oncogene encodes a transmembrane receptor protein of 185kDa, which is structurally related to the epidermal growth factor receptor. Trastuzumab is an antibody that has antigen binding residues of, or derived from, the murine 4D5 antibody (ATCC CRL 10463, deposited with American Type Culture Collection, 12301 Parklawn Drive, Rockville, Md. 20852 under the Budapest Treaty on May 24, 1990). Exemplary humanized 4D5 antibodies include huMAb4D5-1, huMAb4D5-2, huMAb4D5-3, huMAb4D5-4, huMAb4D5-5, huMAb4D5-6, huMAb4D5-7 and huMAb4D5-8 (HERCEPTIN®) as in US 5821337.

Trastuzumab-MCC-DMl may be prepared according to Example 1 of U.S. Application Publication No. 20110165155, for example.

### Pertuzumab Compositions

The Pertuzumab composition comprises a mixture of a main species Pertuzumab antibody, as hereinabove defined, and one or more variants thereof. The preferred embodiment herein of a Pertuzumab main species antibody is one comprising the variable light and variable heavy amino acid sequences in SEQ ID Nos. 7 and 8, and most preferably comprising a light chain amino acid sequence of SEQ ID No. 11, and a heavy chain amino acid sequence of SEQ ID No. 12 (including deamidated and/or oxidized variants of those sequences). In one embodiment, the composition comprises a mixture of the main species Pertuzumab antibody and an amino acid sequence variant thereof comprising an amino-terminal leader extension. Preferably, the amino-terminal leader extension is on a light chain of the antibody variant (e.g. on one or two light chains of the antibody variant). The main species HER2 antibody or the antibody variant may be an full length antibody or antibody fragment (e.g. Fab of F(ab')2 fragments), but preferably both are full length antibodies. The antibody variant herein may comprise an amino-terminal leader extension on any one or more of the heavy or light chains thereof. Preferably, the amino-terminal leader extension is on one or two light chains of the antibody. The amino-terminal leader extension preferably comprises or consists of VHS--. Presence of the amino-terminal leader extension in the composition can be detected by various analytical techniques including, but not limited to, N-terminal sequence analysis, assay for charge heterogeneity (for instance, cation exchange chromatography or capillary zone electrophoresis), mass spectrometry, etc. The amount of the antibody variant in the composition generally ranges from an amount that constitutes the detection limit of any assay (preferably N-terminal sequence analysis) used to detect the variant to an amount less than the amount of the main species antibody. Generally, about 20% or less (e.g. from about 1% to about 15%, for instance from 5% to about 15%) of the antibody molecules in the composition comprise an amino-terminal leader extension. Such percentage amounts are preferably determined using quantitative N-terminal sequence analysis or cation exchange analysis (preferably using a high-resolution, weak cation-exchange column, such as a PROPAC WCX-10™ cation exchange column). Aside from the amino-terminal leader extension variant, further amino acid sequence alterations of the main species antibody and/or variant are contemplated, including but not limited to an antibody comprising a C-terminal lysine residue on one or both heavy chains thereof, a deamidated antibody variant, etc.

Moreover, the main species antibody or variant may further comprise glycosylation variations, non-limiting examples of which include antibody comprising a G1 or G2 oligosaccharide structure attached to the Fc region thereof, antibody comprising a carbohydrate moiety attached to a light chain thereof (e.g. one or two carbohydrate moieties, such as glucose or galactose, attached to one or two light chains of the antibody, for instance attached to one or more lysine residues), antibody comprising one or two non-glycosylated heavy chains, or antibody comprising a sialidated oligosaccharide attached to one or two heavy chains thereof etc.

The composition may be recovered from a genetically engineered cell line, e.g. a Chinese Hamster Ovary (CHO) cell line expressing the HER2 antibody, or may be prepared by peptide synthesis.

For more information regarding exemplary Pertuzumab compositions, see U.S. Pat. Nos. 7,560,111 and 7,879,325 as well as US 2009/0202546A1.

### Formulations of Tratuzumab-MCC-DM1 (T-DM1) and Pertuzumab

Trastuzumab-MCC-DMl and Pertuzumab may be formulated in accordance with standard pharmaceutical practice for use in a therapeutic combination. The pharmaceutical compositions comprise Trastuzumab-MCC-DMl and Pertuzumab, respectively, in association with one or more pharmaceutically acceptable carrier, glidant, diluent, or excipient.

Suitable carriers, diluents and excipients are well known to those skilled in the art and include materials such as carbohydrates, waxes, water soluble and/or swellable polymers, hydrophilic or hydrophobic materials, gelatin, oils, solvents, water and the like. The particular carrier, diluent or excipient used will depend upon the means and purpose for which the compound of the present invention is being applied. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG 400, PEG 300), etc. and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., a compound of the present invention or pharmaceutical composition thereof) or aid in the manufacturing of the pharmaceutical product (i.e., medicament).

The formulations may be prepared using conventional dissolution and mixing procedures. For example, the bulk drug substance (i.e., compound of the present invention or stabilized form of the compound (e.g., complex with a cyclodextrin derivative or other known complexation agent) is dissolved in a suitable solvent in the presence of one or more of the excipients described above. The compound of the present invention is typically formulated into pharmaceutical dosage forms to provide an easily controllable dosage of the drug and to enable patient compliance with the prescribed regimen.

The pharmaceutical composition (or formulation) for application may be packaged in a variety of ways depending upon the method used for administering the drug. Generally, an article for distribution includes a container having deposited therein the pharmaceutical formulation in an appropriate form. Suitable containers are well known to those skilled in the art and include materials such as bottles (plastic and glass), sachets, ampoules, plastic bags, metal cylinders, and the like. The container may also include a tamper-proof assemblage to prevent indiscreet access to the contents of the package. In addition, the container has deposited thereon a label that describes the contents of the container. The label may also include appropriate warnings.

Pharmaceutical formulations may be prepared for various routes and types of administration with pharmaceutically acceptable diluents, carriers, excipients or stabilizers (Remington's Pharmaceutical Sciences (1995) 18th edition, Mack Publ. Co., Easton, PA), in the form of a lyophilized formulation, milled powder, or an aqueous solution. Formulation may be conducted by mixing at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of compound, but may range from about 3 to about 8.

The pharmaceutical formulation is preferably sterile. In particular, formulations to be used for *in vivo* administration must be sterile. Such sterilization is readily accomplished by filtration through sterile filtration membranes.

The pharmaceutical formulation ordinarily can be stored as a solid composition, a lyophilized formulation or as an aqueous solution.

The pharmaceutical formulations of the invention will be dosed and administered in a fashion, i.e., amounts, concentrations, schedules, course, vehicles and route of administration, consistent with good medical practice. Factors for consideration in this context include the particular disorder being treated, the clinical condition of the individual patient, the cause of the disorder, the site of delivery of the agent, the method of administration, the scheduling of administration, and other factors known to medical practitioners.

Acceptable diluents, carriers, excipients and stabilizers are nontoxic to recipients at the dosages and concentrations employed, and include buffers such as phosphate, citrate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl, ethanol, or benzylalcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, including Tween 80, PLURONICS™ or polyethylene glycol (PEG), including PEG400. The active pharmaceutical ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 18th edition, (1995) Mack Publ. Co., Easton, PA. Other examples of drug formulations can be found in Liberman, H. A. and Lachman, L., Eds., Pharmaceutical Dosage Forms, Marcel Decker, Vol 3, 2nd Ed., New York, NY.

The pharmaceutical formulations include those suitable for the administration routes detailed herein. The formulations may conveniently be presented in unit dosage form and may be prepared by any of the methods well known in the art of pharmacy. Techniques and formulations generally are found in Remington's Pharmaceutical Sciences 18th Ed. (1995) Mack Publishing Co., Easton, PA. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Pharmaceutical compositions may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may be a solution or a suspension in a non-toxic parenterally acceptable diluent or solvent, such as a solution in 1,3-butanediol or prepared from a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight:weight). The pharmaceutical composition can be prepared to provide easily measurable amounts for administration. For example, an aqueous solution intended for intravenous infusion may contain from about 3 to 500 µg of the active ingredient per milliliter of solution in order that infusion of a suitable volume at a rate of about 30 mL/hr can occur.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

As a general proposition, the initial pharmaceutically effective amount of Trastuzumab-MCC-DMl administered per dose will be in the range of about 0.3 to 15 mg/kg/day of patient body weight.

A commercial T-DM1 fomulation (KADCYLA®, ado-Trastuzumab emtansine) is a sterile, white to off-white preservative free lyophilized powder in single-use vials. Each vial contains 100 mg or 160 mg ado-Trastuzumab emtansine. Following reconstitution, each single-use vial contains ado-Trastuzumab emtansine (20 mg/mL), polysorbate 20 [0.02% (w/v)], sodium succinate (10 mM), and sucrose [6% (w/v)] with a pH of 5.0 and density of 1.026 g/mL. The resulting solution containing 20 mg/mL adoTrastuzumab emtansine is administered by intravenous infusion following dilution.

A commercial formulation of Pertuzumab (PERJETA®) contains Pertuzumab 420mg/14mL (30mg/mL) in the form of a preservative-free solution for IV infusion.

### Administration of Trastuzumab-DMl (T-DM1) and Pertuzumab

Pharmaceutical compositions of Trastuzumab-MCC-DMl (T-DM1) and Pertuzumab may be administered by any route appropriate to the condition to be treated. Suitable routes include oral, parenteral (including subcutaneous, intramuscular, intravenous, intraarterial, inhalation, intradermal, intrathecal, epidural, and infusion techniques), transdermal, rectal, nasal, topical (including buccal and sublingual), vaginal, intraperitoneal, intrapulmonary and intranasal. Topical administration can also involve the use of transdermal administration such as transdermal patches or iontophoresis devices. For local immunosuppressive treatment, the compounds may be administered by intralesional administration, including perfusing or otherwise contacting the graft with the inhibitor before transplantation. It will be appreciated that the preferred route may vary with for example the condition of the recipient. Where the compound is administered orally, it may be formulated as a pill, capsule, tablet, etc. with a pharmaceutically acceptable carrier, glidant, or excipient. Where the compound is administered parenterally, it may be formulated with a pharmaceutically acceptable parenteral vehicle or diluent, and in a unit dosage injectable form, as detailed below.

### Articles of Manufacture

Articles of manufacture, or "kits", containing Trastuzumab-MCC-DMl and/or Pertuzumab useful for the treatment methods herein are provided. In one embodiment, the kit comprises a container comprising Trastuzumab-MCC-DMl. In another embodiment, the kit comprises Pertuzumab. In a third embodiment, the kit comprises Trastuzumab-MCC-DMl and Pertuzumab. The kit may further comprise a label or package insert, on or associated with the container. The term "package insert" is used to refer to instructions customarily included in commercial packages of therapeutic products, that contain information about the indications, usage, dosage, administration, contraindications and/or warnings concerning the use of such therapeutic products. Suitable containers include, for example, bottles, vials, syringes, blister pack, etc. The container may be formed from a variety of materials such as glass or plastic. The container may hold Trastuzumab-MCC-DMl and/or Pertuzumab or a formulation thereof which is effective for use in a treatment method herein, and may have a sterile access port (for example, the container may be an intravenous solution bag or a vial having a stopper pierceable by a hypodermic injection needle). The label or package insert indicates that the composition is used in a treatment method as described and claimed herein. The article of manufacture may also contain a further container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kit may further comprise directions for the administration of Trastuzumab-MCC-DMl and/or Pertuzumab. For example, if the kit comprises a first composition comprising Trastuzumab-MCC-DMl and a second pharmaceutical formulation, the kit may further comprise directions for the simultaneous, sequential or separate administration of the first and second pharmaceutical compositions to a patient in need thereof.

In another embodiment, the kits are suitable for the delivery of solid oral forms of Trastuzumab-MCC-DMl and/or Pertuzumab, such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one embodiment, a kit may comprise (a) a first container with Trastuzumab-MCC-DMl contained therein; and optionally (b) a second container with Pertuzumab contained therein. Alternatively, or additionally, the kit may further comprise a third container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Where the kit comprises a composition of Trastuzumab-MCC-DMl and Pertuzumab, the kit may comprise a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

One embodiment of an article of manufacture herein comprises an intravenous (IV) bag containing a stable mixture of Pertuzumab and T-DM1 suitable for administration to a cancer patient. Optionally, the mixture is in saline solution; for example comprising about 0.9% NaCl or about 0.45% NaCl. An exemplary IV bag is a polyolefin or polyvinyl chloride infusion bag, e.g. a 250mL IV bag. According to one embodiment of the invention, the mixture includes about 420mg or about 840mg of Pertuzumab and from from about 100 mg to about 160 mg T-DM1.

Optionally, the mixture in the IV bag is stable for up to 24 hours at 5°C or 30°C. Stability of the mixture can be evaluated by one or more assays selected from the group consisting of: color, appearance and clarity (CAC), concentration and turbidity analysis, particulate analysis, size exclusion chromatography (SEC), ion-exchange chromatography (IEC), capillary zone electrophoresis (CZE), image capillary isoelectric focusing (iCIEF), and potency assay.

### EXAMPLES

In order to illustrate the invention, the following examples are included. However, it is to be understood that these examples do not limit the invention and are only meant to suggest a method of practicing the invention.

**TABLE 1**

| LIST OF ABBREVIATIONS | |
|---|---|
| Abbreviation | Definition |
| AC | doxorubicin and cyclophosphamide |
| ADC | antibody-drug conjugate |
| ADCC | antibody-dependent cellular cytotoxicity |
| ADR | adverse drug reaction |
| AE | adverse event |
| AJCC | American Joint Committee on Cancer |
| ALT | alanine aminotransferase |
| ALND | axillary lymph node dissection |
| ANC | absolute neutrophil count |
| AST | aspartate aminotransferase |
| ATA | anti-therapeutic antibodies |
| AUC | area under concentration-time curve |
| BCIRG | Breast Cancer International Research Group |
| bpCR | breast pathologic complete response |
| BSA | body surface area |
| BUN | blood urea nitrogen |
| CHF | congestive heart failure |
| CI | confidence interval |
| CR | complete response |
| CrCl | creatinine clearance |
| CT | computerized tomography |
| CTCAE | Common Terminology Criteria for Adverse Events |
| DCIS | ductal carcinoma in situ |
| DFS | disease-free survival |
| DILI | drug-induced liver injury |
| DM1 | N2'-deacetyl-N2'-(3-mercapto-1-oxopropyl)-maytansine |
| DNA | deoxyribonucleic acid |
| EBC | early breast cancer |
| EC | ethics committee |
| ECG | electrocardiogram |
| ECHO | echocardiogram |
| ECOG | Eastern Cooperative Oncology Group |
| eCRF | electronic case report form |
| EFS | event-free survival |
| EGF | epidermal growth factor |
| EORTC | European Organization for Research and Treatment of Cancer |
| ER | estrogen receptor |
| FDA | Food and Drug Administration |
| FFPE | formalin-fixed paraffin-embedded |
| FNA | fine needle aspiration |
| FPI | first patient in |
| G-CSF | granulocyte colony-stimulating factor |
| H | Trastuzumab (Herceptin®) |
| HBV | hepatitis B virus |
| HCV | hepatitis C virus |
| HER2 | human epidermal growth factor receptor 2 |
| HIPAA | US Health Insurance Portability and Accountability Act of 1996 |
| HIV | human immunodeficiency virus |
| HR | hazard ratio |
| HRQOL | health-related quality of life |
| IB | Investigator's Brochure |
| ICH | International Conference on Harmonization |
| ICF | informed consent form |
| IDFS | invasive disease-free survival |
| IDMC | Independent Data Monitoring Committee |
| IHC | immunohistochemistry |
| ILD | interstitial lung disease |
| IMP | investigational medicine product |
| IND | investigational new drug |
| INR | International Normalized Ratio |
| IRB | Institutional Review Board |
| IRR | infusion-related reaction |
| ISH | in situ hybridization |
| ITT | intent-to-treat |
| IV | intravenous |
| IxRS | interactive response system |
| kg | kilogram |
| LABC | locally advanced breast cancer |
| LCIS | lobular carcinoma in situ |
| LPLV | last patient, last visit |
| LVEF | left ventricular ejection fraction |
| LVSD | left ventricular systolic dysfunction |
| MAPK | mitogen-activated protein kinase |
| MBC | metastatic breast cancer |
| MCC | nonreducible thioether linkage |
| mg | milligram |
| mRNA | messenger ribonucleic acid |
| MUGA | multiple uptake gated acquisition |
| NaCl | sodium chloride |
| NAST | neoadjuvant systemic therapy |
| NCCN | National Comprehensive Cancer Network |
| NCCTG | North Central Cancer Treatment Group |
| NCI CTCAE | National Cancer Institute Common Terminology Criteria for Adverse Events |
| NRH | nodular regenerative hyperplasia |
| NSABP | National Surgical Adjuvant Breast and Bowel Project |
| NYHA | New York Heart Association |
| OS | overall survival |
| P | Pertuzumab (Perjeta®) |
| pCR | pathological complete response |
| PFS | progression-free survival |
| PgR | progesterone receptor |
| PI3K | phosphoinositide 3-kinase |
| PK | pharmacokinetic |
| PO | orally |
| PRO | patient-reported outcome |
| PVC | polyvinyl chloride |
| QLQ | quality of life questionnaire |
| qRT-PCR | quantitative Reverse transcription-polymerase chain reaction |
| q3w | every 3 weeks |
| RCB | residual cancer burden index |
| SAP | statistical analysis plan |
| SBSR | Study Biological Sample Repository |
| SLNB | sentinel lymph node biopsy |
| SOC | system, organ, class |
| SWFI | sterilized water for injection |
| T | docetaxel |
| TCH | docetaxel-carboplatin-Trastuzumab |
| T-DM1 | Trastuzumab emtansine |
| TNM | primary tumor/regional lymph nodes/distant metastases |
| TRIO | Translational Research in Oncology Group |
| tpCR | total pathologic complete response |
| ULN | upper limit of normal |
| WBC | white blood cell |

### Example 1

### Phase III Clinical Study

This study (BO28408 / TRIO021) is a randomized, global, multicenter, open-label, Phase III, two-arm study in treatment-naive patients with operable, locally advanced or inflammatory, centrally assessed HER2-positive EBC whose primary tumors are >2 cm.

### Patients

The patient population includes patients with treatment-naive, operable, locally advanced or inflammatory, centrally confirmed HER2-positive EBC. Thus, the target population for this study includes patients with newly diagnosed primary invasive breast cancer that is HER2-positive (as determined by the central pathology laboratory) and who will be treated with adjuvant systemic chemotherapy following definitive surgery. The HER2 status is tested centrally before randomization. The size of the primary tumor should be >2 cm by at least one radiographic or clinical measurement. The list of all eligibility criteria is included below.

The investigator or the subinvestigator must ensure that only patients who meet the inclusion and exclusion criteria are offered enrollment in the study. The investigator or subinvestigator should also consider all other relevant factors (medical and non-medical), as well as the risks and benefits of the study therapy, when deciding if a patient is an appropriate candidate for the study.

### Inclusion Criteria

Patients must meet the following criteria for study entry:
Signed written informed consent approved by the study site Institutional Review Board (IRB)/Ethical Committee (EC)
Histologically confirmed invasive breast carcinoma with a primary tumor size of >2cm by at least one radiographic or clinical measurement
HER2- positive breast cancer. HER2-positive status is determined based on pretreatment breast biopsy material and is defined for this particular study as an immunohistochemistry (IHC) score of 3+ and/or positive by ISH, prospectively assessed by a central laboratory prior to study enrollment. ISH positivity is defined as a ratio of ≥2 for the number of HER2 gene copies to the number of signals for chromosome 17 copies. A central laboratory will perform both IHC and ISH assays; however, only one positive result is required for eligibility. Paraffin-embedded tumor tissue blocks or partial blocks must be obtained for central confirmation of HER2 eligibility. Only at those sites where a legitimate site regulation applies that makes the submission of blocks impossible, and only after having obtained Sponsor's approval, submission of different material as described in the study specific sampling manual may be accepted.

Patients with multifocal tumors (more than one tumor confined to the same quadrant as the primary tumor) are eligible provided all sampled lesions are centrally confirmed as HER2-positive.
Stage at presentation: cT2-cT4, cN0-cN3, cM0
Known hormone receptor status of the primary tumor
Patient agreement to undergo mastectomy or breast-conserving surgery after neoadjuvant therapy
Willingness and ability to comply with scheduled visits, treatment plans, laboratory tests and other study procedures, including completion of PRO measures
Age ≥18 years
ECOG performance status of 0 or 1
Adequate organ function during screening (within 7 days before first dose) defined as:
- Absolute neutrophil count (ANC) ≥1500 cells/µL
- Platelet count ≥100,000 cells/µL
- Hemoglobin ≥9 g/dL; patients may receive red blood cell transfusions to obtain this level
- Serum creatinine ≤1.5×upper limit of normal (ULN)
- International Normalized Ratio (INR) and (activated) partial thromboplastin time (aPTT /PTT) ≤1.5×ULN
- Aspartate aminotransferase (AST) and alanine aminotransferase (ALT) ≤ULN
- Serum total bilirubin ≤ULN, except for patients with Gilbert's syndrome for whom direct bilirubin should be within the normal range
- Serum alkaline phosphatase ≤ULN
Baseline LVEF ≥55% measured by echocardiogram (ECHO) or multiple-gated acquisition (MUGA)
For women who are not postmenopausal (≥12 months of non-therapy-induced amenorrhea) or surgically sterile (absence of ovaries and/or uterus): agreement to remain abstinent or use single or combined non-hormonal contraceptive methods that result in a failure rate of <1% per year during the treatment period and for at least 7 months after the last dose of study drug
Abstinence is only acceptable if it is in line with the preferred and usual lifestyle of the patient. Periodic abstinence (e.g., calendar, ovulation, symptothermal, or postovulation methods) and withdrawal are not acceptable methods of contraception. Examples of non-hormonal contraceptive methods with a failure rate of <1% per year include tubal ligation, male sterilization, and certain intrauterine devices. Alternatively, two methods (e.g., two barrier methods such as a condom and a cervical cap) may be combined to achieve a failure rate of <1% per year. Barrier methods must always be supplemented with the use of a spermicide.

Negative serum pregnancy test for premenopausal women, and for women who have experienced menopause onset <12 month prior to first dose of therapy
Documentation of the serologies for hepatitis B virus (HBV), including HB surface antigen (HBsAg) and/or total HB core antibody (anti-HBc), and hepatitis C virus (HCV), including HCV antibody testing. The most recent serologic testing must have occurred within 3 months prior to initiation of neoadjuvant therapy. If such testing has not been done, it must be performed during screening. Patients who have positive HBV or HCV serologies without known active disease must meet the eligibility criteria for ALT, AST, total bilirubin, INR, aPTT/PTT, and alkaline phosphatase on at least two consecutive occasions, separated by at least 1 week, within the 28-day screening period. The second of these evaluations must be performed within 3 days prior to the first administration of study drug.

### Exclusion Criteria

Patients who meet any of the following criteria are excluded from study entry:
Stage IV (metastatic) breast cancer
Patients who have received prior anti-cancer therapy for breast cancer except those patients with a history of breast LCIS surgically managed or DCIS treated exclusively with mastectomy. In case of prior history of LCIS/DCIS >5 years must have passed from surgery until diagnosis of current breast cancer
Patients with multicentric (multiple tumors involving more than 1 quadrant) breast cancer Patients with bilateral breast cancer
Patients who have undergone incisional and/or excisional biopsy of primary tumor and/or axillary lymph nodes
Axillary lymph node dissection prior to initiation of neoadjuvant therapy. Patients with clinically negative axilla (by physical examination and radiographic imaging) may undergo a sentinel lymph node biopsy procedure prior to NAST if in keeping with local practice.

Positive sentinel lymph node prior to neoadjuvant therapy
History of concurrent or previously treated non-breast malignancies except for appropriately treated 1) non-melanoma skin cancer and/or 2) in situ carcinomas, including cervix, colon, and skin. A patient with previous invasive non-breast cancer is eligible provided he/she has been disease free for more than 5 years
Treatment with any investigational drug within 28 days prior to randomization
Current (NCI CTCAE) v4.03 Grade ≥2 peripheral neuropathy
Cardiopulmonary dysfunction as defined by any of the following:
- History of NCI CTCAE (Version 4.0) Grade ≥3 symptomatic CHF or NYHA criteria Class ≥ II
- Angina pectoris requiring anti-anginal medication, serious cardiac arrhythmia not controlled by adequate medication, severe conduction abnormality, or clinically significant valvular disease
   High-risk uncontrolled arrhythmias (i.e., atrial tachycardia with a heart rate > 100/min at rest, significant ventricular arrhythmia [ventricular tachycardia], or higher-grade atrioventricular [AV]-block [second degree AV-block Type 2 [Mobitz 2] or third degree AV-block])
- Significant symptoms (Grade ≥ 2) relating to left ventricular dysfunction, cardiac arrhythmia, or cardiac ischemia
- Myocardial infarction within 12 months prior to randomization
- Uncontrolled hypertension (systolic blood pressure >180 mmHg and/or diastolic blood pressure >100mmHg)
- Evidence of transmural infarction on ECG
- Requirement for oxygen therapy
Current severe, uncontrolled systemic disease that may interfere with planned treatment (e.g., clinically significant cardiovascular, pulmonary, or metabolic disease; wound-healing disorders)
Major surgical procedure unrelated to breast cancer or significant traumatic injury within 28 days prior to randomization or anticipation of the need for major surgery during the course of study treatment
Known active liver disease, for example, due to HBV, HCV, autoimmune hepatic disorders, or sclerosing cholangitis
Concurrent, serious, uncontrolled infections or known infection with HIV
Current pregnancy and/or breastfeeding
Known hypersensitivity to study drugs, excipients and/or murine proteins

### Study Design

Patients who have consented and are eligible for the study are randomized to six cycles of one of the following neoadjuvant treatment regimens in a 1:1 ratio:
**Arm** A: Docetaxel (75 mg/m² every 3 weeks [q3w]) and carboplatin (area under concentration-time curve [AUC] 6) and Trastuzumab (8mg/kg loading dose, 6mg/kg maintenance dose q3w) and Pertuzumab (840mg loading dose, then 420mg dose q3w) (docetaxel-carboplatin-Trastuzumab [TCH] + Pertuzumab)
**Arm B:** Trastuzumab emtansine (3.6mg/kg q3w) and Pertuzumab (840mg loading dose, then 420mg dose q3w) (Trastuzumab emtansine+Pertuzumab)

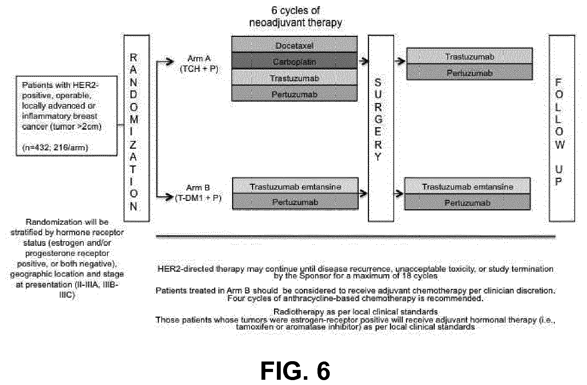

The scheme of the study design is shown in Figure 6, wherein T=docetaxel, C = carboplatin, H = Trastuzumab.

The study will enroll a total of 432 patients, 216 patients per treatment arm, at approximately 110 sites globally.

The patients receive six cycles of neoadjuvant therapy according to their randomization arm. Surgery should not be conducted until at least 14 days after last dose of neoadjuvant therapy. Platelet counts should be checked prior to surgery and should be ≥ 75,000 cells/µL. Surgery should be performed no later than 9 weeks following the last dose of neoadjuvant therapy. All patients randomized to Arm B (Trastuzumab emtansine+Pertuzumab), irrespective of their pCR outcome in the neoadjuvant setting, will additionally be allowed to receive standard cytotoxic therapy in the adjuvant setting; the decision to administer standard cytotoxic therapy and the choice of regimen is at the discretion of the treating physician. Four cycles of anthracycline-based therapy (FAC, FEC or AC) is recommended. Adjuvant treatment is initiated within 9 weeks after the final surgical procedure. HER2-directed therapy is intended to be given as per the randomized arm for 1 year to be consistent with published adjuvant data and global standards. Given the use of therapy in the neoadjuvant and adjuvant setting and possible delays in therapy, the length of HER2-directed therapy (Trastuzumab+Pertuzumab [Arm A] and Trastuzumab emtansine+Pertuzumab [Arm B]) is defined by total number of cycles given. One year of HER2-directed therapy without any delays would encompass 18 cycles; therefore the study plans for administration of 18 cycles of HER2-targeted therapy (inclusive of neoadjuvant and adjuvant therapy).

### Neoadjuvant Phase

Neoadjuvant therapy is administered for a total of six cycles given q3w. In the event of disease progression, unacceptable toxicity, withdrawal of consent, or study termination by the Sponsor, whichever occurs first, neoadjuvant therapy will be discontinued prior to these six cycles. Patients whose neoadjuvant study treatment is discontinued prior to completion of these six cycles and who did not receive non-protocol neoadjuvant therapy will be allowed to receive adjuvant study treatment as per randomization.

Any patient who receives non-protocol therapy prior to surgery will be discontinued from study treatment and will be managed as per local practice.

Patients in Arm B who discontinue Trastuzumab emtansine should also have Pertuzumab discontinued and are considered discontinued from study drug treatment. These patients will be managed as per local practice.

Patients who discontinue Pertuzumab for toxicity may remain on study treatment (Arm A: [TCH only]; Arm B [Trastuzumab emtansine only]).

All patients who discontinue planned study treatment will remain on study for follow-up of secondary and exploratory endpoints unless consent from study participation is withdrawn.

### Surgery

Surgery is performed no later than 6 weeks following the last infusion of neoadjuvant therapy.

Patients should be seen by a surgeon with breast cancer surgery experience prior to initiation of neoadjuvant therapy. The study will evaluate breast conservation rates. Prior to neoadjuvant therapy, the surgeon should evaluate the patient for the surgical procedure that could be conducted based upon the examination at the time of the baseline visit (i.e., in the absence of neoadjuvant therapy would mastectomy or a wide local excision procedure such as segmental/partial mastectomy be required). This baseline assessment should be documented in the electronic case report form (eCRF). The tumor site must be marked with a radiopaque marker under radiographic guidance (e.g., ultrasound) prior to initiation of neoadjuvant therapy.

After completion of neoadjuvant therapy, prior to surgery, the patient should be evaluated for the surgical procedure they would be a candidate to receive based upon their response to therapy (mastectomy or a wide local excision procedure such as segmental/partial mastectomy) and this potential choice should be documented in the eCRF. The selected surgery should also be entered in the eCRF. It is recognized that patients and their surgeons may elect to proceed with a different surgery than they would candidates to receive for a variety of factors (patient preference, risk reduction, etc.). Both the potential surgery based upon response as well as the chosen surgery should be recorded in the eCRF. Surgery should not be conducted until at least 14 days after last dose of neoadjuvant therapy. Platelet counts should be checked prior to surgery and should be ≥ 75,000 cells/µL.

The primary efficacy endpoint, (pCR-ypT0/is, ypN0) will be established via local review following completion of neoadjuvant therapy and surgery.

For patients whose tumor remains inoperable after neoadjuvant treatment, locoregional and/or systemic management is done as per local standard practice. These patients will be withdrawn from study treatment and will remain on study for follow-up of secondary and exploratory endpoints unless they have withdrawn consent from study participation.

Surgical management options for axillary lymph nodes include sentinel lymph node biopsy (SLNB) (prior or after neoadjuvant treatment) and axillary lymph node dissection (ALND) of level I and II lymphatics at the moment of breast surgery. The choice of the axillary procedure will be based on the clinical status of axilla, T stage, and local practice.

### Adjuvant Phase

All patients who have undergone surgery continue to receive the same HER2-directed therapy in the adjuvant phase as was administered in the neoadjuvant phase of the study (Arm A [Trastuzumab+Pertuzumab]; Arm B [Trastuzumab emtansine+Pertuzumab]). Treatment is given so that 18 total cycles of HER2-directed therapy inclusive of therapy given both in the neoadjuvant and adjuvant setting are administered. Patients who discontinue Trastuzumab emtansine because of toxicity that may be attributed to the Trastuzumab component (e.g., hypersensitivity, cardiac toxicity, pneumonitis) may not continue to receive Trastuzumab after discontinuation of Trastuzumab emtansine.

Adjuvant therapy is initiated within 9 weeks after the last surgical procedure. Adjuvant therapy is discontinued in the event of invasive disease recurrence, second primary invasive malignancy, unacceptable toxicity, withdrawal of consent, or study termination by the Sponsor, whichever occurs first. Patients whose adjuvant study treatment is discontinued prior to completion of planned therapy, will still be followed as per protocol for secondary endpoints unless consent to participate is withdrawn.

After surgery, radiotherapy should be administered as clinically indicated. Patients with ER-positive and/or PgR-positive tumors are required to receive adjuvant endocrine therapy (e.g., tamoxifen or aromatase inhibitor) as per local clinical standards.

### Optional Adjuvant Therapy for Arm B

All patients randomized to Arm B (Trastuzumab emtansine+Pertuzumab), regardless of their pCR outcome in the neoadjuvant setting, are additionally be allowed to receive standard cytotoxic therapy in the adjuvant setting; the decision to administer standard cytotoxic therapy and the choice of regimen is at the discretion of the treating physician. Adjuvant anthracycline-based therapy for a minimum of four cycles is recommended (e.g., AC, FAC, FEC). For patients in Arm B for whom chemotherapy is given, adjuvant Trastuzumab should also be initiated with chemotherapy within 9 weeks after last surgical procedure when clinically appropriate. Trastuzumab emtansine and Pertuzumab should not be combined with adjuvant chemotherapy but should be resumed once adjuvant chemotherapy has been completed. Trastuzumab emtansine and Pertuzumab should be resumed within 28 days after completion of optional chemotherapy.

For patients receiving a standard adjuvant chemotherapy regimen, radiation therapy should be delayed until after completion of adjuvant chemotherapy and be initiated within 28 days of completing the adjuvant chemotherapy.

### Length of Study

The primary efficacy endpoint, pCR, is analyzed once all patients have received surgery, approximately 8 months after the last patient has been randomized.

Secondary efficacy endpoints of EFS, IDFS, breast conservation rate and OS are analyzed at a median follow-up time of approximately 36 months from randomization (i.e., when the 50th percentile patient is followed-up for approximately 36 months). When this median follow up time is reached, all patients are contacted for evaluation of secondary endpoints of EFS, IDFS and OS. Descriptive interim analyses of the secondary efficacy endpoints EFS, IDFS and OS may be conducted at/after conducting the primary efficacy analyses and as needed or requested by HA after primary analysis. The total duration of the study is approximately 45 months.

### Efficacy Outcome Measures

### Primary Efficacy Outcome Measures

The primary efficacy outcome measures for this study are as follows:

DFS defined as the time from randomization until the date of the first occurrence of one of the following events:
1. Ipsilateral invasive breast tumor recurrence (i.e., an invasive breast cancer involving the same breast parenchyma as the original primary lesion)
2. Ipsilateral local-regional invasive breast cancer recurrence (i.e., an invasive breast cancer in the axilla, regional lymph nodes, chest wall, and/or skin of the ipsilateral breast)
3. Contralateral or ipsilateral second primary invasive breast cancer
4. Distant recurrence (i.e., evidence of breast cancer in any anatomic site [other than the three sites mentioned above] that has either been histologically confirmed or clinically/radiographically diagnosed as recurrent invasive breast cancer

Death attributable to any cause, including breast cancer, non-breast cancer, or unknown cause.

As described above, the primary efficacy variable is IDFS, defined as the time between randomization and date of first occurrence of an IDFS event. Patients who have not had an event at the time of data analysis will be censored at the date on which they are last known to be alive and event-free, on or before the clinical data cutoff date of the respective analysis.

The log-rank test, stratified by the protocol-defined stratification factors (excluding region) is used to compare IDFS between the two treatment arms. Region is excluded because of the likely loss of power as a result of the potential that some of the strata may have very few patients. The unstratified log-rank test results are also provided for sensitivity analysis. If at the time of analysis it is deemed that the smallest stratum per arm necessary to conduct robust stratified analyses contains < 5 events, unstratified analyses will be used as the primary analysis. The Cox proportional hazards model, stratified by the previously noted stratification factors, excluding region, will be used to estimate the HR between the two treatment arms and its 95% CI. The Kaplan-Meier approach will be used to estimate 3-year IDFS rates and corresponding 95% CIs for each treatment arm.

### Secondary Efficacy Outcome Measures

The secondary efficacy outcome measures for this study are as follows:
IDFS plus second primary non-breast cancer, excluding non-melanoma skin cancers and carcinoma in situ (CIS) of any site
DFS, defined as the time between randomization and the date of the first occurrence of any of the IDFS events described above, second primary non-breast cancer event (excluding non-melanoma skin cancers and CIS of any non-breast site), and contralateral or ipsilateral ductal carcinoma in situ (DCIS)
DRFI, defined as the time between randomization and the first occurrence of distant breast cancer recurrence
OS, defined as the time from randomization to death due to any cause

### Safety Outcome Measures

Clinical and laboratory adverse events will be reported according to the National Cancer Institute Common Terminology Criteria for Adverse Events (NCI CTCAE) v4.03. LVEF will be assessed using either echocardiogram (ECHO) or multiple-gated acquisition (MUGA) scans.

The safety outcome measures for this study are as follows:
Incidence, type, and severity of all adverse events based on NCI CTCAE v4.03
Incidence, type, and severity of serious adverse events
Incidence, type and severity of≥ Grade 3 adverse events
Incidence and type of adverse events leading to dose discontinuation, modification, or delay Cause of death
Abnormal laboratory values
Decrease in LVEF from baseline over time
Cardiac safety outcome measures
   1. Primary cardiac endpoints: cardiac events defined as death from cardiac cause or severe CHF (NYHA Class III or IV) with a decrease in LVEF of≥ 10 percentage points from baseline to an LVEF of< 50%
   2. Secondary cardiac endpoints: other cardiac-related events (e.g., any mild symptomatic CHF [NYHA Class II] associated with a≥ 10% drop in LVEF to < 50%; asymptomatic declines in LVEF requiring dose delay or discontinuation)
Hepatic safety outcome measures
   3. Death from hepatic cause
   4. Severe DILI (Hy's law cases)
   5. NRH
Pulmonary safety outcome measures
   6. Death from pulmonary cause
   7. Pneumonitis and ILD

Secondary endpoints are IDFS plus second primary non-breast cancer, DFS, DRFI (defined in the Efficacy Outcomes Measures section), and OS.

Secondary endpoints are analyzed in a similar manner as the primary endpoint to estimate 3-year event rates (and 5-year survival rate for OS) for each treatment arm and the HR between the two treatment arms with 95% CI. Patients who have not had an event at the time of data analysis are censored at the date on which they are last known to be alive and event-free or prior to the clinical data cutoff date for the respective analysis.

At the end of the trial the Kaplan-Meier approach is used to estimate 5-year IDFS rates and corresponding 95% CIs for each treatment arm using both the overall protocol-defined population and the node-positive subpopulation.

### Patient Reported Outcome (PRO) Measures

The PRO measures for this study are as follows:
HRQoL, including bothersome side-effects of therapy (e.g., peripheral neuropathy, joint/muscle pain, skin problems), and patient functioning as measured using the EORTC QLQ-C30 and the modified breast cancer module QLQ-BR23
Time from first HER2 targeted treatment, ± a taxane, to worsening of global health status/QoL (subscale of the QLQ-C30). The event worsening of global health status/QoL for a given patient is defined as an increase in mean score by 10 points or more at any of the timepoints after initiation of HER2-directed therapy, ± a taxane. Increase in mean score has been defined as being 'moderate' to 'very much' perceived important change from the patient's perspective (Osoba et al. Interpreting the significance of changes in health-related quality-of-life scores. J Clin Oncol 1998; 16(1):139-44).

### Exporatory Biomarker Outcome Measures

The exploratory biomarker outcome measures for this study are the relationship between molecular markers and efficacy and/or safety outcomes. Efficacy outcomes considered for this analysis will include IDFS and OS, as appropriate.

Correlations between biomarker status and efficacy and/or safety will include, but not be limited to, the following:
Level of HER2 mRNA expression assessed by quantitative real-time polymerase chain reaction (qRT-PCR) with efficacy outcome
Status of PIK3CA mutations assessed by PIK3CA allele-specific polymerase chain reaction assay with efficacy outcome
Level of HER2 gene amplification assessed by in situ hybridization (ISH) with efficacy outcome
Level of HER2 protein expression assessed by immunohistochemistry (IHC) with efficacy outcome
Changes in expression levels of biomarker or biomarker panels over time with efficacy outcome

### Dosage, Administration and Compliance

SoC chemotherapy backbone treatments should include three to four cycles of an anthracycline-based regimen. In Arm A, three to four cycles or 12 weeks of taxane are also administered. Administration of HER2-targeted therapy will be up to 1 year (up to 18 cycles). Adjuvant study treatment will be discontinued in the event of invasive disease recurrence, unacceptable toxicity, withdrawal of consent, or study termination by the Sponsor. Patients diagnosed with in situ breast cancer or a second primary cancer not requiring systemic therapy and with no evidence of invasive breast cancer recurrence should continue with adjuvant study treatment, if considered by the investigator to be in the patient's best interest, whenever possible.

### Anthracycline Treatment Phase

Either FEC (Table 2) or AC/EC (Table 3) regimens as described in following subsections may be selected at the discretion of the investigator in this study. Please refer to local prescribing information/institutional guidelines for detailed guidelines on administration, premedications, and dose delays/reductions for toxicities.

**Table 2 FEC**

| **Drug** | **Dose** | **Dosing Interval** | **Planned Duration** |
|---|---|---|---|
| 5-Fluorouracil (F) | 500-600 mg/m² IV bolus or infusion, according to local policy; dose should be capped at 1200 mg for BSA > 2 m² | Day 1 of q3w cycle | 3-4 x |
| Epirubicin (E) | 90-100 mg/m² IV infusion over 15-30 minutes or infuse according to local policy | Day 1 of q3w cycle | 3-4 x |
| Cyclophosphamide (C) | 500-600 mg/m² IV over 30 minutes or infuse according to local policy | Day 1 of q3w cycle | 3-4 x |

| | | | |
|---|---|---|---|
| BSA=body surface area; IV=intravenous; q3w=every 3 weeks; x=cycle. | | | |

**Table 3 AC/EC**

| **Drug** | **Dose** | **Dosing Interval** | **Planned Duration** |
|---|---|---|---|
| Doxorubicin (A) | 60 mg/m² IV over 15-30 minutes or infuse according to local policy | Day 1 q3w cycle or q2w cycle (dose dense) | 4x |
| Or Epirubicin (E) | 90-100 mg/m² IV infusion over 15-30 minutes or infuse according to local policy | Day 1 q3w cycle or q2w cycle (dose dense) | 4x |
| Cyclophosphamide (C) | 500-600 mg/m² IV over 30 minutes or infuse according to local policy | Day 1 q3w cycle or q2w cycle (dose dense) | 4x |

| | | | |
|---|---|---|---|
| IV=intravenous; q2w=every 2 weeks; q3w=every 3 weeks; x=cycle. | | | |

The dose-dense (every 2 weeks [q2w]) AC/EC regimen may be administered with G-CSF support (e.g., pegfilgrastim 6 mg subcutaneously on Day 2 of q2w cycle).

Antiemetic regimens may be used as premedication at the physician's discretion.

### Concurrent Taxane Phase and/or HER2 Targeted Only Phase

Concurrent taxane phase only applies to treatment Arm A (control arm). Trastuzumab plus Pertuzumab must start concurrently with the taxane component of chemotherapy following anthracycline therapy in the control arm. After anthracycline treatment, a minimum interval of 3 weeks from the last dose of anthracycline to initiation of HER2-targeted therapy is required. Prior to commencing the HER2-targeted component of therapy, patients must have a LVEF ≥ 50% and must not have experienced any clinical symptoms suggesting heart failure or asymptomatic LVEF declines by an absolute point of> 15% from baseline. HER2 targeted treatment will continue for up to a total duration of 1 year and be discontinued in the event of invasive disease recurrence, unacceptable toxicity, withdrawal of consent, or study termination by the Sponsor.

A±3-day window is allowed for q3w dosing, and a+3-day window is allowed for qw dosing. This time window does not apply when dose delay is indicated due to toxicities.

### Trastuzumab plus Pertuzumab plus Taxane Treatment

During the taxane concurrent phase, either docetaxel q3w (at 100 mg/m² for three cycles, at 75 mg/m² for four cycles, or start at 75 mg/m² in the first cycle, and escalate to 100 mg/m² if no DLT occurs for a total of three cycles at minimum) or 12 weeks of paclitaxel 80 mg/m² qw will be administered concurrently with Trastuzumab in combination with Pertuzumab. Please refer to local prescribing information/institutional guidelines for detailed guidelines on docetaxel or paclitaxel administration, premedications, and dose delays/reductions for toxicities.

After the concurrent phase, only administration of Trastuzumab plus Pertuzumab will continue for up to a total duration of 1 year (52 weeks; up to 18 cycles).

Trastuzumab will be given at a loading dose of 8 mg/kg and Pertuzumab at 840 mg. For subsequent cycles, Trastuzumab will be given as a maintenance dose of 6 mg/kg and Pertuzumab at 420 mg q3w. The dose of Trastuzumab does not need to be re-calculated unless the body weight has changed by more than ± 10% from baseline. If the patient misses a dose of Trastuzumab for any cycle (i.e., the two sequential administration times are 6 weeks or more apart), a re-loading dose of 8 mg/kg of Trastuzumab should be given. If the patient misses a dose of Pertuzumab for any cycle and the time between doses is 6 weeks or more, a re-loading dose of Pertuzumab (840 mg) should be given. Patients who experience Trastuzumab or Pertuzumab infusion-related symptoms may be pre-medicated with paracetamol and anti-histamines for subsequent infusions.

The sequence of administration for this treatment arm should follow that outlined in Table 4 (sequence as from top to bottom).

**Table 4 Treatment Regimen for Arm 1**

| **Drug** | | **Infusion Period ^{a}** | **Observation Period** | **Planned Duration** |
|---|---|---|---|---|
| Pertuzumab | First dose | 60 minutes | 60 minutes | Up to 18 cycles |
| | Subsequent doses | 30 to 60 minutes according to tolerability | 30 minutes if well tolerated | |
| Trastuzumab ^{b} | First dose | 90 minutes (first dose) | See National prescribing information | Up to 18 cycles |
| | Subsequent doses | 30 to 90 minutes according to tolerability | | |
| Taxane | Docetaxel | 60 minutes | See National prescribing information | 4 cycles |
| | Or Paclitaxel | 30 to 60 minutes | | 12 weeks |

| | | | | |
|---|---|---|---|---|
| ^{a} Infusion period may be longer than described here at the investigator's discretion for patient safety. ^{b} Trastuzumab infusion to start only after observation period for Pertuzumab completed. | | | | |

### Trastuzumab Emansine plus Pertuzumab Treatment

Taxane will not be administered in patients in treatment Arm 2. Trastuzumab emtansine plus Pertuzumab will continue for up to a total duration of 1 year (52 weeks; up to 18 cycles).

Trastuzumab emtansine will be given at a dose of 3.6 mg/kg by IV infusion in combination with Pertuzumab at an initial loading dose of 840 mg IV followed by a maintenance dose of 420 mg IV q3w. The dose of Trastuzumab emtansine does not need to be recalculated unless the body weight has changed by more than±10% from baseline. If the patient misses a dose of Pertuzumab for any cycle and the time between doses is 6 weeks or more, a re-loading dose of Pertuzumab (840 mg) should be given.

Patients who experience Pertuzumab infusion-related symptoms may be pre-medicated with paracetamol and anti-histamines for subsequent infusions.

The sequence of administration for this treatment arm should follow the table below (Table 5) (sequence as from top to bottom).

**Table 5 Treatment Regimen for Arm 2**

| **Drug** | | **Infusion Period** | **Observation Period** | **Planned Duration** |
|---|---|---|---|---|
| Pertuzumab | First dose | 60 minutes | 60 minutes | Up to 18 cycles |
| | Subsequent doses | 30 to 60 minutes according to tolerability | 30 minutes if well tolerated | |
| Trastuzumab emtansine ^{a} | First dose | 90 minutes | 90 minutes | Up to 18 cycles |
| | Subsequent doses | 30 to 90 minutes according to tolerability | 30 minutes if well tolerated | |

| | | | | |
|---|---|---|---|---|
| ^{a} Trastuzumab emtansine infusion to start only after observation period for Pertuzumab completed | | | | |

The foregoing description is considered as illustrative only of the principles of the invention. Further, since numerous modifications and changes will be readily apparent to those skilled in the art, it is not desired to limit the invention to the exact construction and process shown as described above. Accordingly, all suitable modifications and equivalents may be considered to fall within the scope of the invention as defined by the claims that follow.
1. A method for the treatment of breast cancer, comprising
   (i) subjecting a patient with HER2-positive, operable, locally advanced or inflammatory breast cancer to neoadjuvant treatment with a combination of T-DM1 and pertuzumab, in the absence of chemotherapy,
   (ii) removing said breast cancer by definitive surgery; and
   (iii) subjecting said patient to adjuvant treatment with a combination of T-DM1 and Pertuzumab, in the absence of chemotherapy.
2. The method of claim 1, wherein said patient is subjected to adjuvant treatment with a combination of T-DM1 and pertuzumab, in the absence of chemotherapy that comprises a taxane.
3. The method of claim 1, wherein said patient is subjected to adjuvant treatment with a combination of T-DM1 and pertuzumab, in the absence of concurrent chemotherapy.
4. The method of claim 3, wherein the adjuvant treatment comprises chemotherapy prior to and/or following treatment with T-DM1 and pertuzumab.
5. The method of claim 4, wherein the chemotherapy prior to and/or following treatment with T-DM1 and pertuzumab does not comprise a taxane.
6. The method of claim 4, wherein the chemotherapy that is administered comprises anthracycline-based chemotherapy.
7. The method of claim 6, wherein the chemotherapy that is administered further comprises trastuzumab.
8. The method of claim 6, wherein the anthacycline-based therapy comprises one or more of FAC (5-fluoroacil, doxorubicin, cyclophosphamide), FEC (5-fluorouracil, epirubicin and cyclophosphamide) or AC (doxorubicin, cyclophosphamide).
9. The method of any of claims 1-8, wherein said breast cancer is >2 cm in diameter.
10. The method of any of claims 1-8, wherein definitive surgery is performed at least 14 days following the completion of neoadjuvant therapy.
11. The method of claim 10, wherein definitive surgery is performed no later than 9 weeks following the completion of neoadjuvant therapy.
12. The method of any of claims 1-8, wherein the neoadjuvant and adjuvant treatment protocols each comprise infusion of T-DM1 at a dose of 3.6 mg/kg every 3 weeks and infusion of pertuzumab at a loading dose of 840mg and at a dose of 420mg every 3 weeks thereafter.
13. The method of any of the preceding claims, wherein T-DM1 and pertuzumab are administered concurrently.
14. The method of claim 13, wherein T-DM1 and pertuzumab are co-administered.
15. The method of claim 13, wherein T-DM1 and pertuzumab are administered consecutively in either order.
16. The method of claim 13, wherein the administration follows the schedule set forth in Table 5.
17. The method of any of the preceding claims, wherein said treatment increases one or more of complete response (CR), EFS (event-free survival), DFS (disease-free survival), IDFS (invasive diseas-free survival), and OS (overall survival).
16. The method of any of the preceding claims, wherein said treatment increases time to disease progression.
17. The method of any of the preceding claims, wherein neoadjuvant treatment consists essentially of administration of T-DM1 and pertuzumab.
18. The method of any of the preceding claims, wherein neoadjuvant treatment consists of administration of T-DM1 and pertuzumab.
19. The method of any of the preceding claims, wherein adjuvant treatment consists essentially of administration of T-DM1 and pertuzumab.
20. The method of any of the preceding claims, wherein adjuvant treatment consists of administration of T-DM1 and pertuzumab.

## Claims

1. Trastuzumab-MCC-DMl (T-DM1) for use in a method for the neoadjuvant and adjuvant treatment of breast cancer, wherein the method comprises
(i) subjecting a patient with HER2-positive, operable, locally advanced or inflammatory breast cancer to neoadjuvant treatment with a concurrently administered combination of T-DM1 and pertuzumab, wherein said combination is administered in the absence of chemotherapy,
(ii) removing said breast cancer by definitive surgery; and
(iii) subjecting said patient to adjuvant treatment with a concurrently administered combination of T-DM1 and Pertuzumab, wherein said combination is administered in the absence of chemotherapy.

2. T-DM1 for use in a method of claim 1, wherein the adjuvant treatment comprises chemotherapy prior to and/or following treatment with the combination of T-DM1 and pertuzumab.

3. T-DM1 for use in a method of claim 2, wherein the chemotherapy prior to and/or following treatment with the combination of T-DM1 and pertuzumab does not comprise a taxane.

4. T-DM1 for use in a method of claim 2, wherein the chemotherapy that is administered comprises anthracycline-based chemotherapy.

5. T-DM1 for use in a method of claim 4, wherein the chemotherapy that is administered further comprises trastuzumab.

6. T-DM1 for use in a method of claim 4, wherein the anthacycline-based therapy comprises one or more of FAC (5-fluoroacil, doxorubicin, cyclophosphamide), FEC (5-fluorouracil, epirubicin and cyclophosphamide) or AC (doxorubicin, cyclophosphamide).

7. T-DM1 for use in a method of any of claims 1-6, wherein said breast cancer is >2 cm in diameter.

8. T-DM1 for use in a method of any of claims 1-6, wherein the neoadjuvant and adjuvant treatment protocols each comprise infusion of T-DM1 at a dose of 3.6 mg/kg every 3 weeks and infusion of pertuzumab at a loading dose of 840 mg and at a dose of 420 mg every 3 weeks thereafter.

9. T-DM1 for use in a method of any of claims 1-8, wherein T-DM1 and pertuzumab are co-administered.

10. T-DM1 for use in a method of any of claims 1-8, wherein T-DM1 and pertuzumab are administered consecutively in either order.

11. T-DM1 for use in a method of any of claim 1-8, wherein the administration follows the schedule set forth in Table 5.

12. T-DM1 for use in a method of any of the preceding claims, wherein said treatment increases one or more of complete response (CR), EFS (event-free survival), DFS (disease-free survival), IDFS (invasive disease-free survival), and OS (overall survival).

13. T-DM1 for use in a method of any of the preceding claims, wherein said treatment increases time to disease progression.

14. T-DM1 for use in a method of any of the preceding claims, wherein neoadjuvant treatment consists of administration of T-DM1 and pertuzumab.

15. T-DM1 for use in a method of any of the preceding claims, wherein adjuvant treatment consists of administration of T-DM1 and pertuzumab.


SEQUENCE LISTING

<110> GENENTECH, INC.

<120> METHODS OF TREATING EARLY BREAST CANCER WITH TRASTUZUMAB-MCC-DM1
      AND PERTUZUMAB

<130> GNE-0412-WO

<140>
<141>

<150> 61/984,132
<151> 2014-04-25

<160> 16

<170> PatentIn version 3.5

<210> 1
<211> 195
<212> PRT
<213> Homo sapiens

<400> 1
Thr Gln Val Cys Thr Gly Thr Asp Met Lys Leu Arg Leu Pro Ala Ser
1               5                   10                  15

Pro Glu Thr His Leu Asp Met Leu Arg His Leu Tyr Gln Gly Cys Gln
            20                  25                  30

Val Val Gln Gly Asn Leu Glu Leu Thr Tyr Leu Pro Thr Asn Ala Ser
        35                  40                  45

Leu Ser Phe Leu Gln Asp Ile Gln Glu Val Gln Gly Tyr Val Leu Ile
    50                  55                  60

Ala His Asn Gln Val Arg Gln Val Pro Leu Gln Arg Leu Arg Ile Val
65                  70                  75                  80

Arg Gly Thr Gln Leu Phe Glu Asp Asn Tyr Ala Leu Ala Val Leu Asp
                85                  90                  95

Asn Gly Asp Pro Leu Asn Asn Thr Thr Pro Val Thr Gly Ala Ser Pro
            100                 105                 110

Gly Gly Leu Arg Glu Leu Gln Leu Arg Ser Leu Thr Glu Ile Leu Lys
        115                 120                 125

Gly Gly Val Leu Ile Gln Arg Asn Pro Gln Leu Cys Tyr Gln Asp Thr
    130                 135                 140

Ile Leu Trp Lys Asp Ile Phe His Lys Asn Asn Gln Leu Ala Leu Thr
145                 150                 155                 160

Leu Ile Asp Thr Asn Arg Ser Arg Ala Cys His Pro Cys Ser Pro Met
                165                 170                 175

Cys Lys Gly Ser Arg Cys Trp Gly Glu Ser Ser Glu Asp Cys Gln Ser
            180                 185                 190

Leu Thr Arg
        195

<210> 2
<211> 124
<212> PRT
<213> Homo sapiens

<400> 2
Thr Val Cys Ala Gly Gly Cys Ala Arg Cys Lys Gly Pro Leu Pro Thr
1               5                   10                  15

Asp Cys Cys His Glu Gln Cys Ala Ala Gly Cys Thr Gly Pro Lys His
            20                  25                  30

Ser Asp Cys Leu Ala Cys Leu His Phe Asn His Ser Gly Ile Cys Glu
        35                  40                  45

Leu His Cys Pro Ala Leu Val Thr Tyr Asn Thr Asp Thr Phe Glu Ser
    50                  55                  60

Met Pro Asn Pro Glu Gly Arg Tyr Thr Phe Gly Ala Ser Cys Val Thr
65                  70                  75                  80

Ala Cys Pro Tyr Asn Tyr Leu Ser Thr Asp Val Gly Ser Cys Thr Leu
                85                  90                  95

Val Cys Pro Leu His Asn Gln Glu Val Thr Ala Glu Asp Gly Thr Gln
            100                 105                 110

Arg Cys Glu Lys Cys Ser Lys Pro Cys Ala Arg Val
        115                 120

<210> 3
<211> 169
<212> PRT
<213> Homo sapiens

<400> 3
Cys Tyr Gly Leu Gly Met Glu His Leu Arg Glu Val Arg Ala Val Thr
1               5                   10                  15

Ser Ala Asn Ile Gln Glu Phe Ala Gly Cys Lys Lys Ile Phe Gly Ser

20                          25                          30

Leu Ala Phe Leu Pro Glu Ser Phe Asp Gly Asp Pro Ala Ser Asn Thr
        35                          40                          45

Ala Pro Leu Gln Pro Glu Gln Leu Gln Val Phe Glu Thr Leu Glu Glu
    50                          55                          60

Ile Thr Gly Tyr Leu Tyr Ile Ser Ala Trp Pro Asp Ser Leu Pro Asp
65                          70                          75                          80

Leu Ser Val Phe Gln Asn Leu Gln Val Ile Arg Gly Arg Ile Leu His
                        85                          90                          95

Asn Gly Ala Tyr Ser Leu Thr Leu Gln Gly Leu Gly Ile Ser Trp Leu
                100                         105                         110

Gly Leu Arg Ser Leu Arg Glu Leu Gly Ser Gly Leu Ala Leu Ile His
        115                         120                         125

His Asn Thr His Leu Cys Phe Val His Thr Val Pro Trp Asp Gln Leu
    130                         135                         140

Phe Arg Asn Pro His Gln Ala Leu Leu His Thr Ala Asn Arg Pro Glu
145                         150                         155                         160

Asp Glu Cys Val Gly Glu Gly Leu Ala
                        165

<210> 4
<211> 142
<212> PRT
<213> Homo sapiens

<400> 4
Cys His Gln Leu Cys Ala Arg Gly His Cys Trp Gly Pro Gly Pro Thr
1                   5                           10                          15

Gln Cys Val Asn Cys Ser Gln Phe Leu Arg Gly Gln Glu Cys Val Glu
                20                          25                          30

Glu Cys Arg Val Leu Gln Gly Leu Pro Arg Glu Tyr Val Asn Ala Arg
        35                          40                          45

His Cys Leu Pro Cys His Pro Glu Cys Gln Pro Gln Asn Gly Ser Val
    50                          55                          60

Thr Cys Phe Gly Pro Glu Ala Asp Gln Cys Val Ala Cys Ala His Tyr
65                          70                          75                          80

Lys Asp Pro Pro Phe Cys Val Ala Arg Cys Pro Ser Gly Val Lys Pro
                85                  90                  95

Asp Leu Ser Tyr Met Pro Ile Trp Lys Phe Pro Asp Glu Glu Gly Ala
            100                 105                 110

Cys Gln Pro Cys Pro Ile Asn Cys Thr His Ser Cys Val Asp Leu Asp
        115                 120                 125

Asp Lys Gly Cys Pro Ala Glu Gln Arg Ala Ser Pro Leu Thr
    130                 135                 140

<210> 5
<211> 107
<212> PRT
<213> Artificial Sequence

<220>
<221> source
<223> /note="Description of Artificial Sequence: Synthetic
      polypeptide"

<400> 5
Asp Thr Val Met Thr Gln Ser His Lys Ile Met Ser Thr Ser Val Gly
1               5                   10                  15

Asp Arg Val Ser Ile Thr Cys Lys Ala Ser Gln Asp Val Ser Ile Gly
            20                  25                  30

Val Ala Trp Tyr Gln Gln Arg Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln Tyr Tyr Ile Tyr Pro Tyr
                85                  90                  95

Thr Phe Gly Gly Gly Thr Lys Leu Glu Ile Lys
            100                 105

<210> 6
<211> 119
<212> PRT
<213> Artificial Sequence

<220>

<221> source
<223> /note="Description of Artificial Sequence: Synthetic
      polypeptide"

<400> 6
Glu Val Gln Leu Gln Gln Ser Gly Pro Glu Leu Val Lys Pro Gly Thr
1               5                   10                  15

Ser Val Lys Ile Ser Cys Lys Ala Ser Gly Phe Thr Phe Thr Asp Tyr
            20                  25                  30

Thr Met Asp Trp Val Lys Gln Ser His Gly Lys Ser Leu Glu Trp Ile
        35                  40                  45

Gly Asp Val Asn Pro Asn Ser Gly Gly Ser Ile Tyr Asn Gln Arg Phe
    50                  55                  60

Lys Gly Lys Ala Ser Leu Thr Val Asp Arg Ser Ser Arg Ile Val Tyr
65                  70                  75                  80

Met Glu Leu Arg Ser Leu Thr Phe Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

Thr Thr Leu Thr Val Ser Ser
        115


<210> 7
<211> 107
<212> PRT
<213> Artificial Sequence

<220>
<221> source
<223> /note="Description of Artificial Sequence: Synthetic
      polypeptide"

<400> 7

<210> 8
<211> 119
<212> PRT
<213> Artificial Sequence

<220>
<221> source
<223> /note="Description of Artificial Sequence: Synthetic
      polypeptide"

<400> 8

<210> 9
<211> 107
<212> PRT
<213> Homo sapiens

<400> 9
Asp Ile Gln Met Thr Gln Ser Pro Ser Ser Leu Ser Ala Ser Val Gly

1               5                   10                  15

Asp Arg Val Thr Ile Thr Cys Arg Ala Ser Gln Ser Ile Ser Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Ser Leu Pro Trp
                85                  90                  95

Thr Phe Gly Gln Gly Thr Lys Val Glu Ile Lys
            100                 105

<210> 10
<211> 119
<212> PRT
<213> Homo sapiens

<400> 10
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ala Val Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Arg Val Gly Tyr Ser Leu Tyr Asp Tyr Trp Gly Gln Gly
            100                 105                 110

Thr Leu Val Thr Val Ser Ser
        115

<210> 11
<211> 214
<212> PRT
<213> Artificial Sequence

<220>
<221> source
<223> /note="Description of Artificial Sequence: Synthetic
      polypeptide"

<400> 11
Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Val Ser Ile Gly
            20                  25                  30

Val Ala Trp Tyr Gln Gln Lys Pro Gly Lys Ala Pro Lys Leu Leu Ile
        35                  40                  45

Tyr Ser Ala Ser Tyr Arg Tyr Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ile Tyr Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 12
<211> 448
<212> PRT
<213> Artificial Sequence

<220>
<221> source
<223> /note="Description of Artificial Sequence: Synthetic
      polypeptide"

<400> 12
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly

435                 440                 445

<210> 13
<211> 214
<212> PRT
<213> Artificial Sequence

<220>
<221> source
<223> /note="Description of Artificial Sequence: Synthetic
      polypeptide"

<400> 13
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser

195                 200                 205

Phe Asn Arg Gly Glu Cys
    210

<210> 14
<211> 449
<212> PRT
<213> Artificial Sequence

<220>
<221> source
<223> /note="Description of Artificial Sequence: Synthetic
      polypeptide"

<400> 14
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro

180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210> 15
<211> 217
<212> PRT
<213> Artificial Sequence

<220>
<221> source
<223> /note="Description of Artificial Sequence: Synthetic
      polypeptide"

<400> 15
Val His Ser Asp Ile Gln Met Thr Gln Ser Pro Ser Ser Leu Ser Ala
1               5                   10                  15

Ser Val Gly Asp Arg Val Thr Ile Thr Cys Lys Ala Ser Gln Asp Val
            20                  25                  30

Ser Ile Gly Val Ala Trp Tyr Gln Gln Lys Pro Gly Lys Ala Pro Lys
        35                  40                  45

Leu Leu Ile Tyr Ser Ala Ser Tyr Arg Tyr Thr Gly Val Pro Ser Arg
    50                  55                  60

Phe Ser Gly Ser Gly Ser Gly Thr Asp Phe Thr Leu Thr Ile Ser Ser
65                  70                  75                  80

Leu Gln Pro Glu Asp Phe Ala Thr Tyr Tyr Cys Gln Gln Tyr Tyr Ile
                85                  90                  95

Tyr Pro Tyr Thr Phe Gly Gln Gly Thr Lys Val Glu Ile Lys Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys
    210                 215

<210> 16
<211> 449
<212> PRT
<213> Artificial Sequence

<220>
<221> source
<223> /note="Description of Artificial Sequence: Synthetic
      polypeptide"

<400> 16
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Tyr
            20                  25                  30

Thr Met Asp Trp Val Arg Gln Ala Pro Gly Lys Gly Leu Glu Trp Val
        35                  40                  45

Ala Asp Val Asn Pro Asn Ser Gly Gly Ser Ile Tyr Asn Gln Arg Phe
    50                  55                  60

Lys Gly Arg Phe Thr Leu Ser Val Asp Arg Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asn Leu Gly Pro Ser Phe Tyr Phe Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys